# EUROPEAN PATENT SPECIFICATION

(11) **EP 1 716 257 B1**
(45) Date of publication and mention of the grant of the patent: **20.10.2010**
(21) Application number: 05715271.2
(22) Date of filing: 08.02.2005
(51) Int. Cl.: C12Q 1/70

(54) **NEW PRIMERS AND PROBES FOR THE DETECTION OF PARVOVIRUS B19**
NEUE PRIMER UND SONDEN ZUM NACHWEIS VON PARVOVIRUS B19
NOUVELLES AMORCES ET SONDES POUR LA DÉTECTION DU PARVOVIRUS B19

(30) Priority: 10.02.2004 EP 04002854
(43) Date of publication of application: 02.11.2006
(60) Divisional application: 10161839.5
(73) Proprietor: Roche Diagnostics GmbH, 68305 Mannheim (DE); F.HOFFMANN-LA ROCHE AG, 4070 Basel (CH)
(72) Inventor: SCHORLING, Stefan, 82377 Penzberg (DE)
(74) Representative: Herren, Barbara
(86) International application number: PCT/EP2005/001243
(87) International publication number: WO 2005/075686

(56) References cited:
- WO-A-99/28439
- SCHMIDT I ET AL: "Parvovirus B19 DNA in plasma pools and plasma derivatives." VOX SANGUINIS. NOV 2001, vol. 81, no. 4, November 2001 (2001-11), pages 228-235, XP002288712 ISSN: 0042-9007
- HARDER T C ET AL: "New LightCycler PCR for rapid and sensitive quantification of parvovirus B19 DNA guides therapeutic decision-making in relapsing infections" JOURNAL OF CLINICAL MICROBIOLOGY 2001 UNITED STATES, vol. 39, no. 12, 2001, pages 4413-4419, XP002288713 ISSN: 0095-1137
- AUBIN J T ET AL: "Large-scale screening for human parvovirus B19 DNA by PCR: Application to the quality control of plasma for fractionation" VOX SANGUINIS, vol. 78, no. 1, February 2000 (2000-02), pages 7-12, XP002288714 ISSN: 0042-9007
- MUELLER J ET AL: "Development and validation of a real-time PCR assay for routine testing of blood donations for parvovirus B19 DNA." INFUSION THERAPY AND TRANSFUSION MEDICINE, vol. 29, no. 5, September 2002 (2002-09), pages 254-258, XP008032754 ISSN: 1424-5485
- GRUBER FRANZ ET AL: "Quantitation of viral DNA by real-time PCR applying duplex amplification, internal standardization, and two-color fluorescence detection" APPLIED AND ENVIRONMENTAL MICROBIOLOGY, vol. 67, no. 6, June 2001 (2001-06), pages 2837-2839, XP002288716 ISSN: 0099-2240

### SEQUENCE LISTING

<110> Roche Diagnostics GmbH
<120> New primers and probes for the detection of parvovirus B19
<130> 22398 WO
<150> EP 04002854.0
   <151> 2004-02-10
<160> 17
<170> PatentIn version 3.2
<210> 1
   <211> 349
   <212> DNA
   <213> Parvovirus B19
<400> 1
   gtggtgaaag ctctgaagaa ctcagtgaaa gcagcttttt taacctcatc accccaggcg     60
   cctggaacac tgaaaccccg cgctctagta cgcccatccc cgggaccagt tcaggagaat    120
   catttgtcgg aagcccagtt tcctccgaag ttgtagctgc atcgtgggaa gaagccttct    180
   acacaccttt ggcagaccag tttcgtgaac tgttagttgg ggttgattat gtgtgggacg    240
   gtgtaagggg tttacctgtg tgttgtgtgc aacatattaa caatagtggg ggagggttgg    300
   gactttgtcc ccattgcatt aatgtagggg cttggtataa tggatggaa                349
<210> 2
   <211> 95
   <212> DNA
   <213> Parvovirus B19
<400> 2
   gtggtgaaag ctctgaagaa ctcagtgaaa gcagcttttt taacctcatc accccaggcg 60
   cctggaacac tgaaaccccg cgctctagta cgccc 95
<210> 3
   <211> 29
   <212> DNA
   <213> Parvovirus B19
<400> 3
   tcaaccccaa ctaacagttc acgaaactg 29
<210> 4
   <211> 32
   <212> DNA
   <213> Parvovirus B19
<400> 4
   ttccatccat tataccaagc ccctacatta at 32
<210> 5
   <211> 55
   <212> DNA
   <213> Parvovirus B19
<400> 5
   tccccgggac cagttcagga gaatcatttg tcggaagccc agtttcctcc gaagt 55
<210> 6
   <211> 35
   <212> DNA
   <213> Parvovirus B19
<400> 6
   cagtggtggt gaaagctctg aagaactcag tgaaa 35
<210> 7
   <211> 34
   <212> DNA
   <213> Parvovirus B19
<400> 7
   ctggaacact gaaaccccgc gctctagtac gccc 34
<210> 8
   <211> 29
   <212> DNA
   <213> Parvovirus B19
<400> 8
   tcaaccccaa ctaacagttc acgaaactg 29
<210> 9
   <211> 24
   <212> DNA
   <213> Parvovirus B19
<400> 9
   aaatttccat ccattatacc aagc 24
<210> 10
   <211> 37
   <212> DNA
   <213> Parvovirus B19
<400> 10
   ccccgggacc agttcaggag aatcatttgt cggaagc 37
<210> 11
   <211> 26
   <212> DNA
   <213> Parvovirus B19
<400> 11
   ccccgggacc agttcaggag aatcat 26
<210> 12
   <211> 20
   <212> DNA
   <213> Parvovirus B19
<400> 12
   gtggtgaaag ctctgaagaa 20
<210> 13
   <211> 16
   <212> DNA
   <213> Parvovirus B19
<400> 13
   gaaaccccgc gctcta 16
<210> 14
   <211> 18
   <212> DNA
   <213> Parvovirus B19
<400> 14
   aaaccccgcg ctctagta 18
<210> 15
   <211> 20
   <212> DNA
   <213> Parvovirus B19
<400> 15
   gaaaccccgc gctctagtac 20
<210> 16
   <211> 20
   <212> DNA
   <213> Parvovirus B19
<400> 16
   ttccatccat tataccaagc 20
<210> 17
   <211> 20
   <212> DNA
   <213> Parvovirus B19
<400> 17
   cccaactaac agttcacgaa 20

## Description

### Field of the invention

The present invention relates to new primers and probes for the detection of parvovirus B19 as well as to kits containing them. Further, the invention relates to methods wherein the new primers and probes can be used, in particular homogeneous polymerase chain reaction methods. Further, the invention relates to uses of the new primers and probes.

### Background of the invention

Parvovirus B19 infection is a common childhood illness which usually runs a mild course in immunocompetent individuals, producing a characteristic rash known as erythema infectiosum or fifth disease (Anderson M.J. et al., Lancet 1 (1983) 1378). Infection may be complicated by severe arthralgia or a transient aplastic crisis in individuals suffering from chronic hemolytic disease (J. R. et al., Lancet 1 (1981) 664-665). Congenital anemia and vasculitis have also been described (Cohen B., BMJ 311 (1995) 1549-1552). More recently the virus has been associated with hepatitis and myocarditis (Yoto Y. et al., Lancet 347 (1996) 868-9; Enders G. et al., Clin Infect Dis 26 (1998) 355-358; Dux S. et al., Dtsch Med Wochenschr 127 (2002) 1584-1588). Following maternal infection in pregnancy the virus may be transmitted to the fetus, causing hydrops, spontaneous abortion or intrauterine death (Enders E: Infections of the fetus and the neonate other than rubella. Topley & Wilson's Microbiology and Microbial Infections. Edited by Collier L. London, Edward Arnold, 1998, pp. 873-915). Besides transmission via the respiratory route, parvovirus B19 infection may also occur through contaminated blood and blood products (Brown K. E., Baillieres Best Pract Res Clin Haematol 13 (2000) 245-259). The latter has been recognized by the US Food and Drug Administration, resulting in a proposal for parvovirus B19 nucleic acid testing (NAT) to be regarded as in-process testing rather than donor screening.

In the field of molecular diagnostics, the detection of target nucleic acids with the polymerase chain reaction (PCR) plays an important role. The routine screening of blood banks for the presence of Human Immunodeficiency Virus (HIV), or Hepatitis-B (HBV) or C Virus (HCV) is an example for the large-scale application of PCR-based diagnostics. Automated systems for PCR-based analysis often make use of real-time detection of product amplification during the PCR process. Key to such methods is the use of modified oligonucleotides carrying reporter groups or labels. The detection of DNA amplification products generated by a PCR process can, on the one hand, be accomplished in separate working steps. These may involve the characterisation of amplified fragments with respect to their electrophoretic mobility and/or the analysis of denatured amplification products attached to a solid support using a hybridisation probe.

On the other hand, the detection of DNA amplification products can be done in a so-called "homogeneous" assay system. A "homogeneous" assay system comprises reporter molecules or labels which generate a signal while the target sequence is amplified. An example for a "homogeneous" assay system is the TaqMan^{®} system that has been detailed in US 5,210,015, US 5,804,375 and US 5,487,972. Briefly, the method is based on a double-labelled probe and the 5'-3' exonuclease activity of Taq DNA polymerase. The probe is complementary to the target sequence to be amplified by the PCR process and is located between the two PCR primers during each polymerisation cycle step. The probe has two fluorescent labels attached to it One is a reporter dye, such as 6-carboxyfluorescein (FAM), which has its emission spectra quenched by energy transfer due to the spatial proximity of a second fluorescent dye, 6-carboxy-tetramethyl-rhodamine (TAMRA). In the course of each amplification cycle, the Taq DNA polymerase in the process of elongating a primed DNA strand displaces and degrades the annealed probe, the latter due to the intrinsic 5'-3' exonuclease activity of the polymerase. The mechanism also frees the reporter dye from the quenching activity of TAMRA. As a consequence, the fluorescent activity increases with an increase in cleavage of the probe, which is proportional to the amount of PCR product formed. Accordingly, amplified target sequence is measured detecting the intensity of released fluorescence label. Another example for "homogeneous" assay systems are provided by the formats used in the LightCycler® instrument (see e.g. US 6,174,670), some of them sometimes called "kissing probe" formats. Again, the principle is based on two interacting dyes which, however, are characterised in that the emission wavelength of a donor-dye excites an acceptor-dye by fluorescence resonance energy transfer. The COBAS^{®} AmpliPrep instrument (Roche Diagnostics GmbH, D-68305 Mannheim, Germany) was recently introduced to expand automation by isolating target sequences using biotinylated sequence-specific capture probes along with streptavidin-coated magnetic particles (Jungkind D., J Clin Virol 20 (2001) 1-6; Stelzl E. et al., J Clin Microbiol 40 (2002) 1447-1450). It has lately been joined by an additional versatile tool, the Total Nucleic Acid Isolation (TNAI) Kit (Roche Diagnostics). This laboratory-use reagent allows the generic, not sequence-specific isolation of all nucleic acids from plasma and serum on the COBAS^{®} AmpliPrep instrument based essentially on the method developed by Boom R. et al., J Clin Microbiol 28 (1990) 495-503.

Assay systems for parvovirus B19 are disclosed in Japanese unexamined patent publication No. 147986/1995 or by Schorling, S. et al., J. Mol. Diagn. 6 (2004), 37-41. Primers for the detection of parvovirus B19 from the VP1 or VP2 region are disclosed in US 6,274,307. The cloning of VP1 and VP2 are disclosed in JP 04088985. A probe for parvovirus RA-1 is disclosed in EP 238 893 and probes for parvovirus B19 in WO 01/06019. The NS1 gene and probes thereto are disclosed in EP 783 580. PCR based detection of Parvovirus B19 is described in RU2146372. A nucleic acid sequence of a specific erythrovirus is described in WO 99/28439. WO 03/002753 describes a diagnostic assay for parvovirus B19. WO 02/00924 describes parvoviral phospholipase A2. A method for determining large quantities of parvovirus B19 is described in US 6,183,999. Testing for parvovirus B19 is disclosed in WO 01/14593. DNA control constructs are disclosed in WO 02/096925. Parvovirus-like particles are described in WO 91/04330. JP 11221099 describes PCR amplification of parvovirus B19. A method for treating parvovirus B19 infections is disclosed in US 6,268,349. Autonomous parvovirus B19 gene delivery vehicles are described in US 5,585,254 and corresponding US patents and WO 00/24917.

The sequence of parvovirus B19 is described in Shade, R. O. et al., J Virol 58 (1986) 921-936 and the analysis of the genome is described by Cotmore S. F. et al., J. Virol. 60 (1986) 548-557 and Ozawa K. et al., J. Virol. 62 (1988) 2508-2511. A detection method for parvovirus B19 is described in Sato K. et al., J Clin Microbiol 38 (2000) 1241-1243, Cubie H. A. et al., Mol Cell Probes 9 (1995), 59-66, Jordan J. A. et al., Mol. Diagn. 1 (1996), 321-328, Vassias I. et al., J. Virol. Meth. 44 (1993) 329-338, and Carriere C. et al., J Virol Methods 44 (1993), 221-234, and Holloway B. et al., Nucleic Acids Res 21 (1993), 3905-3906. The VP1 region is analysed by Dorsch S. et al., J. Gen. Virol. (2001) 82, 191-199 and Takahashi N. et al., FEBS Lett. 450 (1999) 289-293. The NS1 region is analysed by Hicks K. E. et al., Arch Virol. 141 (1996), 1319-1327. The sequence variability among different parvovirus B19 isolates is described in Hemauer A. et al., J. Gen. Virol. (1996) 77, 1781-1785, Umene K. and Nunoue T., J. Gen. Virol. 76 (1995) 2645-2651, Erdman D. D. et al., J Gen Virol 77 (1996), 2767-2774 and Astell et al., J. Gen. Virol. 68 (1987) 885-893, and Turton J. et al., Epidemiol Infect 105 (1990) 197-201.

WO99/28439 describes certain primers and probes suitable for the detection of erythrovirus V9 and B19. US 6,395,472 discloses nucleic oligomer primers or probes useful for detection of TT virus. Schmidt et al. (Vox Sanguinis (2001) 81, 228-235) disclose the detection of parvovirus B19 in plasma pools and plasma derivatives.

### Summary of the invention

Because of the clinical significance of parvovirus B19, there is a need for further primers and probes that can detect the virus in biological samples.

Therefore, the invention provides a method for the detection of a target nucleic acid comprising the nucleic acid sequence of parvovirus B19 in a sample comprising the steps of
(a) providing a sample suspected to contain the target nucleic acid
(b) providing a pair of primers comprising a first and a second primer whereby the first primer consists of nucleic acid sequence SEQ ID No:15 and whereby the second primer consists of nucleic acid sequence SEQ ID NO:17,
(c) amplifying the target nucleic acid,
(d) detecting the amplified target nucleic acid of step (c).

The invention further provides a method for the detection of a target nucleic acid comprising the nucleic acid sequence of parvovirus B19 in a sample comprising the steps of
(a) providing a sample suspected to contain the target nucleic acid,
(b) providing a pair of primers comprising a first and a second primer,
(c) amplifying the target nucleic acid,
(d) contacting the sample with a probe under conditions for binding the probe to the target nucleic acid,
(e) detecting the binding product between the target nucleic acid and the probe as an indication of the presence of the target nucleic acid
   characterized in that
   the first primer consists of nucleic acid sequence SEQ ID NO:15, and whereby the second primer consists of nucleic acid sequence SEQ ID NO:17 and
   the probe consists of at least 12 contiguous nucleotides of the nucleic acid sequence SEQ ID NO: 5 or a complementary sequence thereof.

Further disclosed herein is an oligonucleotide whereby the nucleic acid sequence of the oligonucleotide is selected from the nucleic acid sequences SEQ ID NO: 12 to 15, from the nucleic acid sequences 10 or 11 or a complementary sequence thereof or from the complementary sequence of the nucleic acid sequences 16 or 17.

In another embodiment of the invention, a pair of primers is provided comprising a first and a second primer whereby the nucleic acid sequence of the first primer is SEQ ID NO: 15 and whereby the nucleic acid sequence of the second primer is SEQ ID NO:17**.**

The oligonucleotides or the pair of primers according to the invention can be used in a hybridisation reaction with a complementary nucleic acid. In another embodiment of the invention, the oligonucleotides or the pair of primers according to the invention can be used as a primer, probe or capture probe.

The invention further provides a kit comprising a template dependent DNA polymerase, nucleotides and a pair of primers according to the invention.

### Detailed description of the invention

Conventional techniques of molecular biology and nucleic acid chemistry, which are within the skill of the art, are explained in the literature. See, for example, Sambrook J. et al., Molecular Cloning: A Laboratory Manual, Cold Spring Harbor Laboratory Press, Cold Spring Harbor, New York, 1989, Gait, M.J., ed., 1984; Nucleic Acid Hybridization, Hames, B.D., and Higgins, S.J., eds., 1984; and a series, Methods in Enzymology, Academic Press, Inc., all of which are incorporated herein by reference. All patents, patent applications, and publications mentioned herein, both supra and infra, are incorporated herein by reference.

Before nucleic acids may be analysed in one of the above-mentioned assays, they have to be isolated or purified from biological samples containing complex mixtures of different components. Often, for the first steps, processes are used which allow the enrichment of the nucleic acids. To release the contents of cells or viral particles, they may be treated with enzymes or with chemicals to dissolve, degrade or denature the cellular walls or the viral particles. This process is commonly referred to as lysis. The resulting solution containing such lysed material is referred to as lysate. A problem often encountered during the lysis is that other enzymes degrading the component of interest, e.g. desoxyribonucleases or ribonucleases degrading nucleic acids, come into contact with the component of interest during lysis. These degrading enzymes may also be present outside the cells or may have been spatially separated in different cellular compartiments before the lysis and come now into contact with the component of interest. Other components released during this process may be e.g. endotoxins belonging to the family of lipopolysaccharides which are toxic to cells and can cause problems for products intended to be used in human or animal therapy.

There are a variety of means to tackle this problem mentioned-above. It is common to use chaotropic agents as e.g. guanidinium thiocyanate or anionic, cationic, zwitterionic or non-ionic detergents when nucleic acids are intended to be set free. It is also an advantage to use proteases which rapidly degrade these enzymes or unwanted proteins. However, this may produce another problem as the said substances or enzymes can interfere with reagents or components in subsequent steps.

Enzymes which can be advantageously used in such lysis or sample preparation processes mentioned-above are enzymes which cleave the amide linkages in protein substrates and which are classified as proteases, or (interchangeably) peptidases (see Walsh, 1979, Enzymatic Reaction Mechanisms. W. H. Freeman and Company, San Francisco, Chapter 3). Proteases which have been used in the prior art are e.g. alkaline proteases (WO 98/04730) or acid proteases (US 5,386,024). The protease which is widely used in the prior art for sample preparation for the isolation of nucleic acids is proteinase K from *Tritirachium album* (see e.g. Sambrook J. et al., Molecular Cloning: A Laboratory Manual, Cold Spring Harbor Laboratory Press, Cold Spring Harbor, New York, 1989) which is active around neutral pH and belongs to a family of proteases known to the person skilled in the art as subtilisins.

In the next steps of the sample preparation which follow on the lysis step, the component of interest is further enriched. If the non-proteinaceous components of interest are e.g. nucleic acids, they are normally extracted from the complex lysis mixtures before they are used in a probe-based assay.

There are several methods for the extraction of nucleic acids:
- sequence-dependent or biospecific methods as e.g.:
   - affinity chromatography
   - hybridisation to immobilised probes
- sequence-independent or physico-chemical methods as e.g.:
   - liquid-liquid extraction with e.g. phenol-chloroform
   - precipitation with e.g. pure ethanol
   - extraction with filter paper
   - extraction with micelle-forming agents as cetyl-trimethyl-ammonium-bromide
   - binding to immobilised, intercalating dyes, e.g. acridine derivatives
   - adsorption to silica gel or diatomic earths
   - adsorption to magnetic glass particles (MGP) or organo silane particles under chaotropic conditions

Particularly interesting for extraction purposes is the adsorption of nucleic acids to a glass surface although other surfaces are possible. Many procedures for isolating nucleic acids from their natural environment have been proposed in recent years by the use of their binding behavior to glass surfaces. If unmodified nucleic acids are the target, a direct binding of the nucleic acids to a material with a silica surface is preferred because among other reasons the nucleic acids do not have to be modified and even native nucleic acids can be bound. These processes are described in detail by various documents. In Vogelstein B. et al., Proc. Natl. Acad. USA 76 (1979) 615-9, for instance, a procedure for binding nucleic acids from agarose gels in the presence of sodium iodide to ground flint glass is proposed. The purification of plasmid DNA from bacteria on glass dust in the presence of sodium perchlorate is described in Marko M. A. et al., Anal. Biochem. 121 (1982) 382-387. In DE-A 37 34 442, the isolation of single-stranded M13 phage DNA on glass fiber filters by precipitating phage particles using acetic acid and lysis of the phage particles with perchlorate is described. The nucleic acids bound to the glass fiber filters are washed and then eluted with a methanol-containing Tris/EDTA buffer. A similar procedure for purifying DNA from lambda phages is described in Jakobi R et al., Anal. Biochem. 175 (1988) 196-201. The procedure entails the selective binding of nucleic acids to glass surfaces in chaotropic salt solutions and separating the nucleic acids from contaminants such as agarose, proteins or cell residue. To separate the glass particles from the contaminants, the particles may be either centrifuged or fluids are drawn through glass fiber filters. This is a limiting step, however, that prevents the procedure from being used to process large quantities of samples. The use of magnetic particles to immobilize nucleic acids after precipitation by adding salt and ethanol is more advantageous and described e.g. in Alderton R. P. et al., S., Anal. Biochem. 201 (1992) 166-169 and PCT GB 91/00212. In this procedure, the nucleic acids are agglutinated along with the magnetic particles. The agglutinate is separated from the original solvent by applying a magnetic field and performing a wash step. After one wash step, the nucleic acids are dissolved in a Tris buffer. This procedure has a disadvantage, however, in that the precipitation is not selective for nucleic acids. Rather, a variety of solid and dissolved substances are agglutinated as well. As a result, this procedure can not be used to remove significant quantities of any inhibitors of specific enzymatic reactions that may be present. Magnetic, porous glass is also available on the market that contains magnetic particles in a porous, particular glass matrix and is covered with a layer containing streptavidin. This product can be used to isolate biological materials, e.g., proteins or nucleic acids, if they are modified in a complex preparation step so that they bind covalently to biotin. Magnetizable particular adsorbents proved to be very efficient and suitable for automatic sample preparation. Ferrimagnetic and ferromagnetic as well as superparamagnetic pigments are used for this purpose. The most preferred MGPs and methods using magnetic glass particles are those described in WO 01/37291 which is incorporated by reference herein.

After the purification or isolation of the nucleic acids including the target nucleic acid from their natural surroundings, the target nucleic acid may be detected. Therefore, in one embodiment of the invention, a method for the detection of a target nucleic acid comprising the nucleic acid sequence of parvovirus B19 in a sample is provided comprising the steps of
(a) providing a sample suspected to contain the target nucleic acid (b) providing a pair of primers comprising a first and a second primer whereby the first primer consists of nucleic acid sequence SEQ ID NO:15, and whereby the second primer consists of nucleic acid sequence SEQ ID NO:17,
(c) amplifying the target nucleic acid,
(d) detecting the amplified target nucleic acid of step (c).

Preferably, the method does not comprise the step of providing the sample suspected to contain the target nucleic acid. Therefore, in one embodiment of the invention, a method is provided for the detection of a target nucleic acid comprising the nucleic acid sequence of parvovirus B19 in a sample comprising the steps of
(a) amplifying the target nucleic acid in a sample suspected to contain the target nucleic acid in the presence of a pair of primers comprising a first and a second primer whereby the first primer consists of the nucleic acid sequence SEQ ID NO:15, and whereby the second primer consists of at least contiguous 12 nucleotides of a nucleic acid sequence selected from the complementary sequence of the nucleic acid sequences SEQ ID NO: 17, and
(b) detecting the amplified target nucleic acid of step (a).

As is known in the art, a "nucleoside" is a base-sugar combination. The base portion of the nucleoside is normally a heterocyclic base. The two most common classes of such heterocyclic bases are the purines and the pyrimidines.

"Nucleotides" are "nucleosides" that further include a phosphate group covalently linked to the sugar portion of the nucleoside. For those "nucleosides" that include a pentofuranosyl sugar, the phosphate group can be linked to either the 2', 3' or 5' hydroxyl moiety of the sugar. A "nucleotide" is the "monomeric unit" of an "oligonucleotide", more generally denoted herein as an "oligomeric compound", or a "polynucleotide", more generally denoted as a "polymeric compound". Another general expression therefor is desoxyribonucleic acid (DNA) and ribonucleic acid (RNA).

According to the invention, an "oligomeric compound" is a compound consisting of "monomeric units" which may be "nucleotides" alone or "non-natural compounds" (see below), more specifically "modified nucleotides" (or "nucleotide analogs") or "non-nucleotide compounds", alone or combinations thereof. "Oligonudeotides" and "modified oligonucleotides" (or "oligonucleotide analogs") are subgroups of "oligomeric compounds" in the context of the invention.

In the context of this invention, the term "oligonucleotide" refers to "polynucleotides" formed from a plurality of "nucleotides" as the "monomeric unit", i.e. an "oligonucleotide" belongs to a specific subgroup of a "oligomeric compound" or "polymeric compound" of ribonucleic acid (RNA) or deoxyribonucleic acid (DNA) with "monomeric units". The phosphate groups are commonly referred to as forming the internucleoside backbone of the "oligonucleotide". The normal linkage or backbone of RNA and DNA is a 3' to 5' phosphodiester linkage.

"Oligonucleotides" and "modified oligonucleotides" (see below) according to the invention may be synthesized as principally described in the art and known to the expert in the field. Methods for preparing oligomeric compounds of specific sequences are known in the art, and include, for example, cloning and restriction of appropriate sequences and direct chemical synthesis. Chemical synthesis methods may include, for example, the phosphotriester method described by Narang S. A. et al., Methods in Enzymology 68 (1979) 90-98, the phosphodiester method disclosed by Brown E. L., et al., Methods in Enzymology 68 (1979) 109-151, the phosphoramidite method disclosed in Beaucage et al., Tetrahedron Letters 22 (1981) 1859, the H-phosphonate method disclosed in Garegg et al., Chem. Scr. 25 (1985) 280-282 and the solid support method disclosed in US 4,458,066.

As said above, a "nucleic acid" as well as the "target nucleic acid" is a polymeric compound of "nucleotides" as known to the expert skilled in the art. It is used herein to denote a "nucleic acid" in a sample which should be analyzed, i.e. the presence, non-presence or amount thereof in a sample should be determined. Therefore, in other words the "nucleic acid" is the target and can therefore be also denoted as "target nucleic acid". For example, if it has to be determined whether blood contains parvovirus B19, the "target nucleic acid" is the nucleic acid of the Parvovirus B19. The term "complementary (nucleic acid) sequence of a nucleic acid sequence" as used herein means that the complementary (nucleic acid) sequence referred to is exactly the (inverse) complement of the nucleic acid sequence.

The term "primer" is used herein as known to the expert skilled in the art and refers to "oligomeric compounds" primarily to "oligonucleotides" but also to "modified oligonucleotides" that are able to "prime" DNA synthesis by a template-dependent DNA polymerase, i.e. the 3'-end of the e.g. oligonucleotide provides a free 3'-OH group whereto further "nucleotides" may be attached by a template-dependent DNA polymerase establishing 3' to 5' phosphodiester linkage whereby desoxynucleoside triphosphates are used and whereby pyrophosphate is released. Therefore, there is - except for the intended function - no fundamental difference between a "primer", an "oligonucleotide" or a "probe" according to the invention. As used herein, the singular forms "a," "an" and "the" include both singular and plural references unless the content clearly dictates otherwise.

As used herein, the term "comprising" when placed before the recitation of steps in a method means that the method encompasses one or more steps that are additional to those expressly recited, and that the additional one or more steps may be performed before, between, and/or after the recited steps. For example, a method comprising steps a, b, and c encompasses a method of steps a, b, x, and c, a method of steps a, b, c, and x, as well as a method of steps x, a, b, and c. Furthermore, the term "comprising" when placed before the recitation of steps in a method does not (although it may) require sequential performance of the listed steps, unless the content clearly dictates otherwise. For example, a method comprising steps a, b, and c encompasses, for example, a method of performing steps in the order of steps a, c, and b, the order of steps c, b, and a, and the order of steps c, a, and b, etc.

The amplification is performed preferably with the polymerase chain reaction which specifically amplifies target nucleic acids to detectable amounts. Other possible amplification reactions are the Ligase Chain Reaction (LCR; Wu D. Y. and Wallace R B., Genomics 4 (1989) 560-69; and Barany F., Proc. Natl. Acad. Sci. USA 88 (1991)189-193); Polymerase Ligase Chain Reaction (Barany F., PCR Methods and Applic. 1 (1991) 5-16); Gap-LCR (WO 90/01069); Repair Chain Reaction (EP 0439182 A2), 3SR (Kwoh D. Y. et al., Proc. Natl. Acad. Sci. USA 86 (1989) 1173-1177; Guatelli J.C., et al., Proc. Natl. Acad. Sci. USA 87 (1990) 1874-1878; WO 92/08808), and NASBA (US 5,130,238). Further, there are strand displacement amplification (SDA), transciption mediated amplification (TMA), and Qβ-amplification (for a review see e.g. Whelen A. C. and Persing D. H., Annu. Rev. Microbiol. 50 (1996) 349-373; Abramson R. D. and Myers T. W., Curr Opin Biotechnol 4 (1993) 41-47).

Suitable DNA detection methods are known to the expert in the field and are described in standard textbooks as Sambrook J. et al., Molecular Cloning: A Laboratory Manual, Cold Spring Harbor Laboratory Press, Cold Spring Harbor, New York, 1989 and Ausubel F. et al.: Current Protocols in Molecular Biology 1987, J. Wiley and Sons, NY. There may be also further purification steps before the DNA detection step is carried out as e.g. a precipitation step. The detection methods may include but are not limited to the binding or intercalating of specific dyes as ethidiumbromide which intercalates into the double-stranded DNA and changes its fluorescence thereafter. The purified DNA may also be separated by electrophoretic methods optionally after a restriction digest and visualized thereafter. There are also probe-based assays which exploit the oligonucleotide hybridisation to specific sequences and subsequent detection of the hybrid. It is also possible to sequence the DNA after further steps known to the expert in the field. The preferred template-dependent DNA polymerase is Taq polymerase.

A preferred embodiment of the invention are therefore the above-described purification method followed by a determination or detection step or purification methods followed by an amplification and determination or detection step. In another embodiment of the invention, a method is provided for the detection of a target nucleic acid comprising the nucleic acid sequence of parvovirus B19 in a sample comprising the steps of
(a) providing a sample suspected to contain the target nucleic acid,
(b) providing a pair of primers comprising a first and a second primer,
(c) amplifying the target nucleic acid,
(d) contacting the sample with a probe under conditions for binding the probe to the target nucleic acid,
(e) detecting the binding product between the target nucleic acid and the probe as an indication of the presence of the target nucleic acid
   characterized in that
   the first primer consists of the nucleic acid sequence SEQ ID NO: 15, and whereby the second primer consists of the nucleic acid sequence SEQ ID NO: 17, and
   the probe consists of at least 12 contiguous nucleotides of the nucleic acid sequence SEQ ID NO: 5 or a complementary sequence thereof.

Preferably, the method does not comprise the step of providing the sample suspected to contain the target nucleic acid. Therefore, in another embodiment of the invention, a method is provided for the detection of a target nucleic acid comprising the nucleic acid sequence of parvovirus B19 in a sample comprising the steps of
(a) amplifying the target nucleic acid in a sample suspected to contain the target nucleic acid in the presence of a pair of primers comprising a first and a second primer,
(b) contacting the sample of step a) with a probe under conditions for binding the probe to the target nucleic acid,
(c) detecting the binding product between the target nucleic acid and the probe as an indication of the presence of the target nucleic acid
characterized in that
the first primer consists of the nucleic acid sequence SEQ ID NO:15, and whereby the second primer consists of at least contiguous 12 nucleotides of a nucleic acid sequence selected from the complementary sequence of the nucleic acid sequences SEQ ID No:17, and
the probe consists of at least 12 contiguous nucleotides of the nucleic acid sequence SEQ ID NO: 5 or a complementary sequence thereof.

The term "probe" refers to synthetically or biologically produced nucleic acids (DNA or RNA) which, by design or selection, contain specific nucleotide sequences that allow them to hybridize under defined predetermined stringencies specifically (i.e., preferentially) to "target nucleic acids". A "probe" can be identified as a "capture probe" meaning that it "captures" the target nucleic acid so that it can be separated from undesirable materials which might obscure its detection. Once separation is accomplished, detection of the captured "target nucleic acid" can be achieved using a suitable procedure. "Capture probes" are often already attached to a solid phase.

In the method according to the invention, the probe consists preferably of at least 12 contiguous nucleotides of the nucleic acid sequence SEQ ID NO: 10 or a complementary sequence thereof. Preferably, the probe consist of at least 15 or 18 contiguous nucleotides of the nucleic acid sequences of the invention. More preferably, the probe has the nucleic acid sequence SEQ ID NO: 11 or a complementary sequence thereof.

The method according to the invention may be performed in a format for the use in the LightCycler^{®} instrument which is described in US 6,174,670. This format comprises amplification and detection whereby the latter uses the detection of the fluorescence for the detection of the binding product between a pair of probes and the target nucleic acid. These formats apply the fluorescent resonance energy transfer technology (see, for example, US Patent Nos. 4,996,143, 5,565,322, 5,849,489, and 6,162,603) and are based on the fact that when a donor and a corresponding acceptor fluorescent label are positioned within a certain distance of each other, energy transfer takes place between the two fluorescent labels that can be visualized or otherwise detected and/or quantitated. As used herein, two probes, each containing a fluorescent label, whereby at least one thereof is an oligonucleotide according to the invention, can hybridize to an amplification product at particular positions determined by the complementarity of the probes to the target nucleic acid. The fluorescent label according to the invention of the oligonucleotide according to the invention may be a donor or acceptor fluorescent label. Upon hybridization of the probes to the amplification product at the appropriate positions, a FRET signal is generated. Fluorescent analysis can be carried out using, for example, a photon counting epifluorescent microscope system (containing the appropriate dichroic mirror and filters for monitoring fluorescent emission at the particular range), a photon counting photomultiplier system, or a fluorometer. Excitation to initiate energy transfer can be carried out with an argon ion laser, a high intensity mercury (Hg) arc lamp, a fiber optic light source, or other high intensity light source appropriately filtered for excitation in the desired range. As used herein with respect to donor and corresponding acceptor fluorescent labels, "corresponding" refers to an acceptor fluorescent label having an excitation spectrum that overlaps the emission spectrum of the donor fluorescent label. Accordingly, efficient non-radiative energy transfer can be produced there between. The preferred fluorescent label is fluorescein as the donor fluorescent label, whereby the acceptor fluorescent label is rhodamine, however, preferred is a cyanine dye, preferably Cy5 as described in US 6,174,670.

"Labels", often referred to as "reporter groups", are generally groups that make a nucleic acid, in particular the "oligomeric compound" or the "modified oligonucleotide" according to the invention, as well as any nucleic acids bound thereto distinguishable from the remainder of the liquid, i.e. the sample (nucleic acids having attached a "label" can also be termed labeled nucleic acid binding compounds, labeled probes or just probes). Preferred labels according to the invention are fluorescent labels, which are e.g. fluorescent dyes as a fluorescein dye, a rhodamine dye, a cyanine dye, and a coumarin dye.

As used herein, "fluorescence resonance energy transfer relationship" and similar terms refer to adjacent hybridization of an "oligonucleotide" labeled with a "donor fluorescent label" and another "oligomeric compound" labeled with an "acceptor fluorescent label" to a "target nucleic acid" such that the "donor fluorescent label" can transfer resonance energy to the "acceptor fluorescent label" such that the "acceptor fluorescent label" produces a measurable fluorescence emission. If the "donor fluorescent label" and "acceptor fluorescent label" are spaced apart by too great a distance, then the "donor fluorescent label" cannot transfer resonance energy to the "acceptor fluorescent label" such that the "acceptor fluorescent label" emits measurable fluorescence, and hence the "donor fluorescent label" and "acceptor fluorescent label" are not in resonance energy transfer relationship.

In a preferred embodiment of the invention, the probe carries a label. Preferably, an additional probe carrying a label is contacted with the sample in step d) so that a pair of probes consisting of a first and a second probe is contacted with the sample in step d).

In a preferred embodiment of the invention, a method according to the invention is provided wherein said amplifying step c) in the method according to the invention comprises contacting the sample with the said pair of primers to produce an amplification product if the target nucleic acid is present in said sample, wherein said hybridizing step d) comprises contacting said sample with the pair of probes, wherein the members of said pair of probes hybridize to said amplification product within no more than five nucleotides of each other, wherein the first probe of said pair of probes is labeled with a donor fluorescent label and wherein the second probe of said pair of probes is labeled with a corresponding acceptor fluorescent label; and detecting the binding product between the target nucleic acid and the pair of probes in step e) by detecting the presence or absence of fluorescence resonance energy transfer between said donor fluorescent label of said first probe and said acceptor fluorescent label of said second probe, wherein the presence of fluorescence resonance energy transfer is indicative of the presence of the target nucleic acid in the sample, and wherein the absence of fluorescence resonance energy transfer is indicative of the absence of the target nucleic acid in the sample.

Therefore, in an embodiment of the invention, a method for detecting the presence or absence of a target nucleic acid comprising the nucleic acid sequence of parvovirus B 19 in a sample is provided, comprising the steps of performing at least one cycling step, wherein a cycling step comprises an amplifying step and a hybridizing step, wherein said amplifying step comprises contacting said sample with primers, whereby a primer is an oligonucleotide according to the invention, to produce an amplification product if target nucleic acid is present in said sample, wherein said hybridizing step comprises contacting said sample with the pair of probes, wherein the members of said pair of probes hybridize to said amplification product within no more than five nucleotides of each other, wherein a first probe of said pair of probes is labeled with a donor fluorescent label and wherein a second probe of said pair of probes is labeled with a corresponding acceptor fluorescent label, wherein a probe may be an oligonucleotide according to the invention; and detecting the presence or absence of fluorescence resonance energy transfer between said donor fluorescent label of said first probe and said acceptor fluorescent label of said second probe, wherein the presence of FRET is indicative of the presence of the target nucleic acid in the sample, and wherein the absence of FRET is indicative of the absence of the target nucleic acid in the sample.

In another preferred embodiment of the invention, a method for detecting a target nucleic acid comprising the nucleic acid sequence of parvovirus B19 in a sample is provided, comprising the steps of amplifying the nucleic acid by polymerase chain reaction in the presence of two nucleic acid probes, whereby a probe may be an oligonucleotide according to the invention, that hybridize to adjacent regions of the target nucleic acid, one of said probes being labeled with an acceptor fluorescent label and the other probe labeled with a donor fluorescent label of a fluorescence energy transfer pair such that upon hybridization of the two probes with the target nucleic acid, the donor and acceptor fluorescent labels are within 25 nucleotides of one another, said polymerase chain reaction comprising the steps of adding a thermostable polymerase, nucleotides and primers, whereby a primer may be an oligonucleotide according to the invention, for the target nucleic acid to the sample and thermally cycling the sample between at least a denaturation temperature and an elongation temperature; exciting the biological sample with light at a wavelength absorbed by the donor fluorescent label and detecting fluorescent emission from the fluorescence energy transfer pair.

In another preferred embodiment of the invention, a method for the detection of a target nucleic acid comprising the nucleic acid sequence of parvovirus B19 in sample is provided comprising the steps of amplifying the nucleic acid by polymerase chain reaction in the presence of two nucleic acid probes, whereby a probe may be an oligonucleotide according to the invention, that hybridize to adjacent regions of the nucleic acid, one of said probes being labeled with an acceptor fluorescent label and the other probe labeled with donor fluorescent label of a fluorescence energy transfer pair such that upon hybridization of the two probes with the target nucleic acid, the donor and acceptor fluorescent labels are within 25 nucleotides of one another, said polymerase chain reaction comprising the steps of adding a thermostable polymerase, nucleotides and primers, whereby a primer may be an oligonucleotide according to the invention, for the target nucleic acid to the sample and thermally cycling the sample between at least a denaturation temperature and an elongation temperature; exciting the sample with light at a wavelength absorbed by the donor label and monitoring temperature dependent fluorescence from the fluorescence energy transfer pair.

In a preferred embodiment of the method of the invention, the format used in the TaqMan^{®} assay is contemplated. This format comprises amplification and detection whereby the latter uses the detection of the fluorescence for the detection of the binding product the probe and the target nucleic add. Therefor, the probe, which may be an oligonucleotide according to the invention, comprises a label, which is preferably a fluorescent label, preferably fluorescein. The probe may further comprise other fluorescent labels wherein the emission wavelengths of one of the fluorescent labels overlaps the absorption wavelengths of another of the fluorescent labels. Preferably, the probe further comprises a second fluorescent label acting as a quenching agent, that quenches the fluorescence emission of the fluorescent label, which can be fluorescein. Preferably the quenching agent is a fluorescent rhodamine or cyanine. The quenching agent can also be a non-fluorescent compound or dye as dabcyl ("Dark quencher"). The probe cannot be extended enzymatically to be used as probe in the TaqMan^{®} format as principally set out in US 5,210,015, US 5,478,972, or US 5,804,375. Preferably, the monomeric unit at the 3'-end of the oligomeric compound is a 2',3'-dideoxynucleotide or a 3'-phosphorylated nucleotide. In consequence for the format used in the TaqMan^{®} assay, in the determination step of the method, the spatial relationship between the fluorescent label and the second label, i.e. the quenching agent, subsequent to hybridization is altered, preferably by exonuclease hydrolysis of a template-dependent DNA polymerase, preferably the Taq-Polymerase, of the probe whereby release of label occurs as a result of exonuclease hydrolysis. Therefore, in the method according to the invention, the target nucleic in step c) is amplified with a template-dependent DNA polymerase. The degree of hybridization between the oligomeric compound according to the invention and the nucleic acid is determined by the quantity of label that is released from the probe subsequent to hybridization. Therefore it is a preferred embodiment of the invention, that in step (d) the degree of hybridization is determined by the quantity of label that is released from the probe hybridized to the nucleic acid by exonuclease hydrolysis by the template-dependent DNA polymerase.

Therefore, more preferably, the probe, which may be an oligonucleotide according to the invention carries a first and a second label. In the most preferred embodiment, in step (e) the degree of hybridization or the binding product of the probe and the target nucleic acid is determined by the quantity of the first or second fluorescent label that is released from the probe hybridized to the target nucleic acid by exonuclease hydrolysis by the template-dependent DNA polymerase.

In a very preferred embodiment of the invention related in more detail to the TaqMan^{®} assay format, a method for the detection of a target nucleic acid comprising the nucleic acid sequence of parvovirus B19 in a sample is provided comprising the steps of
(a) contacting a sample comprising single-stranded nucleic acids with and a probe containing a first and second fluorescent label, and whereby said probe contains a sequence complementary to a second region of the same target nucleic acid sequence strand, but not including the nucleic acid sequence defined by the primers to create a mixture of duplexes during hybridization conditions, wherein the duplexes comprise the target nucleic acid annealed to the primers and to the probe such that the 3' end of the first primer is upstream of the 5' end of the probe,
(b) maintaining the mixture of step (a) having a 5' to 3' nuclease activity under conditions sufficient to permit the 5' to 3' nuclease activity of the polymerase to cleave the annealed probe and release labelled fragments; and
(c) detecting and/or measuring the release of labelled fragments.

For the above-described method, the nucleic acids can be present in double-stranded or single-stranded form whereby the double-stranded nucleic acids are denatured, i.e. made single-stranded, before the method is performed by heating, i.e. thermal denaturing.

In another preferred embodiment, a primer and/ or the probe may be chemically modified, i.e. the primer and/ or the probe comprise a modified nucleotide or a non-nucleotide compound. The probe or the primer is then a modified oligonucleotide.

"Modified nucleotides" (or "nucleotide analogs") differ from a natural "nucleotide" by some modification but still consist of a base, a pentofuranosyl sugar, a phosphate portion, base-like, pentofuranosyl sugar-like and phosphate-like portion or combinations thereof. For example, a "label" may be attached to the base portion of a "nucleotide" whereby a "modified nucleotide" is obtained. A natural base in a "nucleotide" may also be replaced by e.g. a 7-desazapurine whereby a "modified nucleotide" is obtained as well. The terms "modified nucleotide" or "nucleotide analog" are used interchangeably in the present application. A "modified nucleoside" (or "nucleoside analog") differs from a natural nucleoside by some modification in the manner as outlined above for a "modified nucleotide" (or a "nucleotide analog").

A "non-nucleotide compound" is different from a natural "nucleotide" but is in the sense of this invention still capable - similar to a "nucleotide" - of being a "monomeric unit" of an "oligomeric compound". Therefore, a "non-nucleotide compound" has to be capable of forming an "oligomeric compound" with "nucleotides". Even "non-nucleotide compounds" may contain a base-like, pentofuranosyl sugar-like or a phosphate-like portions, however, not all of them are present at the same time in a "non-nucleotide compound".

A "modified oligonucleotide" (or "oligonucleotide analog") belongs to another specific subgroup of the "oligomeric compounds", that possesses one or more "nucleotides", one or more "non-nucleotide compounds" or "modified nucleotides" as "monomeric units". Thus, the terms "modified oligonucleotide" (or "oligonucleotide analog") refers to structures that function in a manner substantially similar to "oligonucleotides" and are used interchangeably throughout the application. From a synthetical point of view, a "modified oligonucleotide" (or a "oligonucleotide analog") can be for example made by chemical modification of "oligonucleotides" by appropriate modification of the phosphate backbone, ribose unit or the nucleotide bases (Uhlmann and Peyman, Chemical Reviews 90 (1990) 543; Verma S., and Eckstein F., Annu. Rev. Biochem. 67 (1998) 99-134). Representative modifications include phosphorothioate, phosphorodithioate, methyl phosphonate, phosphotriester or phosphoramidate inter-nucleoside linkages in place of phosphodiester inter-nucleoside linkages; deaza or aza purines and pyrimidines in place of natural purine and pyrimidine bases, pyrimidine bases having substituent groups at the 5 or 6 position; purine bases having altered substituent groups at the 2, 6 or 8 positions or 7 position as 7-deazapurines; sugars having substituent groups at, for example, their 2' position; or carbocyclic or acyclic sugar analogs. Other modifications consistent with the spirit of this invention are known to those skilled in the art. Such "modified oligonucleotides" (or "oligonucleotide analogs") are best described as being functionally interchangeable with, yet structurally different from, natural "oligonucleotides" (or synthetic "oligonucleotides" along natural lines). In more detail, exemplary modifications are disclosed in Verma S., and Eckstein F., Annu. Rev. Biochem. 67 (1998) 99-134 or WO 02/12263. In addition, modification can be made wherein nucleoside units are joined through groups that substitute for the internucleoside phosphate or sugar phosphate linkages. Such linkages include those disclosed in Verma S., and Eckstein F., Annu. Rev. Biochem. 67 (1998) 99-134. When other than phosphate linkages are utilized to link the nucleoside units, such structures have also been described as "oligonucleosides".

Another preferred embodiment is related to multiplex detection of various target nucleic acids, preferably different viruses. Therefore, in a preferred embodiment of the invention a method according to the invention is provided wherein other target nucleic acids are detected in the same reaction. Preferably, the other target nucleic acids comprise nucleic acid from hepatitis A virus, hepatitis B virus, hepatitis C virus, west nile virus, the Cytomegalovirus (CMV), the human immunodeficiency virus or bacterial pathogens responsible for bacterial infections as e.g. Neisseria gonorrhoae or chlamydial infections.

Further disclosed herein is an oligonucleotide is provided whereby the nucleic acid sequence of the oligonucleotide is selected from the nucleic acid sequences SEQ ID NO: 12 to 15, from the nucleic acid sequences 10 or 11 or a complementary sequence thereof or from the complementary sequence of the nucleic acid sequences 16 or 17. In a preferred embodiment of the invention, an oligonucleotide is provided which comprises a modified nucleotide or a non-nucleotide compound.

In another embodiment of the invention, a pair of primers is provided comprising a first and a second primer whereby the nucleic acid sequence of the first primer is SEQ ID NO: 15 and whereby the nucleic acid sequence of the second primer is SEQ ID NO:17.

The pair of primers according to the invention can be used in a hybridisation reaction with a complementary nucleic acid. In another embodiment of the invention, the oligonudeotides according to the invention are used as a primers.

In another embodiment a kit of parts is contemplated by the invention whereby the kit contains a template-dependent polymerase having 3' to 5' exonucleolytic activity, preferably the Taq Polymerase, nucleotides and a pair of primers according to the invention. In another embodiment of the invention, a kit is provided comprising a template dependent DNA polymerase, nucleotides and a pair of primers according to the invention.

Such kits known in the art further comprise plastics ware which can be used during the amplification procedure as e.g. microtitre plates in the 96 or 384 well format or just ordinary reaction tubes manufactured e.g. by Eppendorf, Hamburg, Germany and all other reagents for carrying out the method according to the invention.

In another embodiment of the invention, the kit contains further reagents for isolating the nucleic acid. Therefore, the kit can additionally contain a material with an affinity to nucleic acids, preferably the material with an affinity to nucleic acids comprises a material with a silica surface. Preferably, the material with a silica surface is a glass. Most preferably, the material with an affinity to nucleic acids is a composition comprising magnetic glass particles as described in WO 96/41811 or WO 01/37291. The kit can further or additionally comprise a lysis buffer containing e.g. chaotropic agents, detergents or alcohols or mixtures thereof which allows the lysis of cells and separately a protease, e.g. proteinase K, for the digestions of unwanted proteins. These components of the kit according to the invention may be provided separately in tubes or storage containers. Depending on the nature of the components, these may be even provided in a single tube or storage container. The kit may further or additionally comprise a washing solution which is suitable for the washing step of the magnetic glass particles when DNA or RNA is bound thereto. This washing solution may contain ethanol and/ or chaotropic agents in a buffered solution or solutions with an acidic pH without ethanol and/ or chaotropic agents as described above. Often the washing solution or other solutions are provided as stock solutions which have to be diluted before use. The kit may further or additionally comprise an eluent or elution buffer, i.e. a solution or a buffer (e.g. 10 mM Tris, 1 mM EDTA, pH 8.0) or pure water to elute the DNA or RNA bound to the magnetic glass particles. Further, additional reagents or buffered solutions may be present which can be used for the purification process of a nucleic acid, i.e. DNA or RNA.

A preferred embodiment of the present invention is to use the method or the kit of the present invention in automatable methods as e.g. described in WO 99/ 16781. Automatable method means that the steps of the method are suitable to be carried out with an apparatus or machine capable of operating with little or n o external control or influence by a human being. Automatized method means that the steps of the automatable method are carried out with an apparatus or machine capable of operating with little or no external control or influence by a human being. Only the preparation steps for the method may have to be done by hand, e.g. the storage containers have to filled up and put into place, the choice of the samples has to be done by a human being and further steps known to the expert in the field, e.g. the operation of the controlling computer. The apparatus or machine may e.g. add automatically liquids, mix the samples or carry out incubation steps at specific temperatures. Typically, such a machine or apparatus is a robot controlled by a computer which carries out a program in which the single steps and commands are specified. Preferred automatized methods are those which are carried out in a high-throughput format which means that the methods and the used machine or apparatus are optimized for a high-throughput of samples in a short time. In another embodiment of the invention the methods or the kits according to the present invention are used in semi-automatized process which means that some reaction steps may have to be done manually. In a preferred embodiment of the invention, a suspension containing MGPs according to the present invention is taken from a storage container and partial volumes are added to different reaction vessels. Reaction vessels may be reaction tubes made from plastics eventually in mictrotitreplate format contain 96 or 384 or more wells where a reaction can be carried out. However, these vessels may be made from other material e.g. from steel.

In preferred embodiments of the invention the kit according to the invention is used for research, bioanalytics or diagnostics. In preferred embodiments according to the invention the kit according to the invention or the method according to the invention is used in a high-throughput format, i.e. in an automatized method which allows the analysis of a high number of different samples in a very short time.

The following examples, references, sequence listing and figures are provided to aid the understanding of the present invention, the true scope of which is set forth in the appended claims. It is understood that modifications can be made in the procedures set forth.

### Description of the Figures

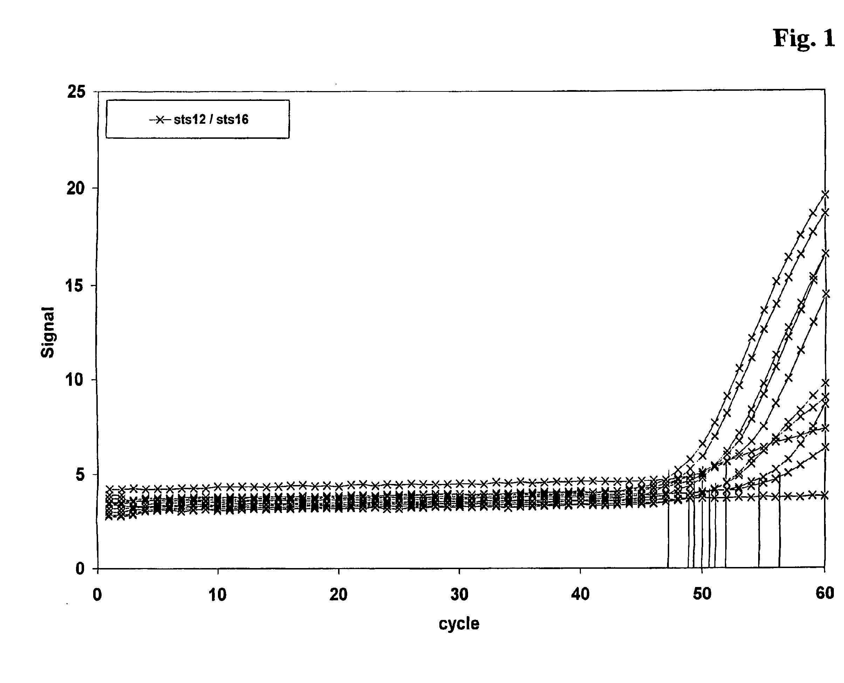

- **Figure 1**: Kinetic PCR growth curves for the primer combination STS12/16 along with probe STS15 basing on an eluate derived from a 1000 IU/mL sample. The vertical lines indicate when the growth curves cross the threshold, in other words, when an unambiguous and specific signal is initially detectable.

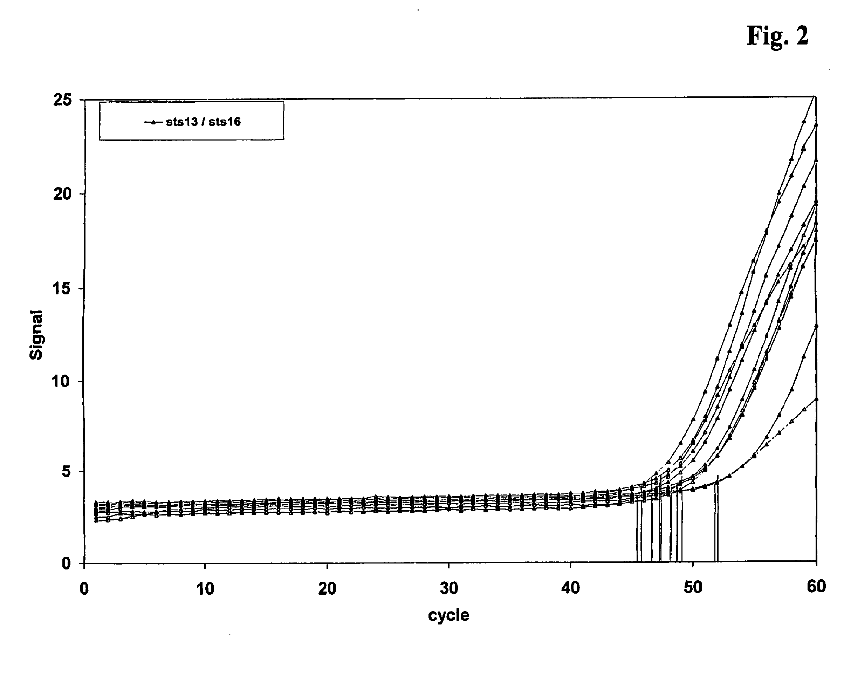

- **Figure 2**: Kinetic PCR growth curves for the primer combination STS13/16 along with probe STS15 basing on an eluate derived from a 1000 IU/mL sample. The vertical lines indicate when the growth curves cross the threshold, in other words, when an unambiguous and specific signal is initially detectable.

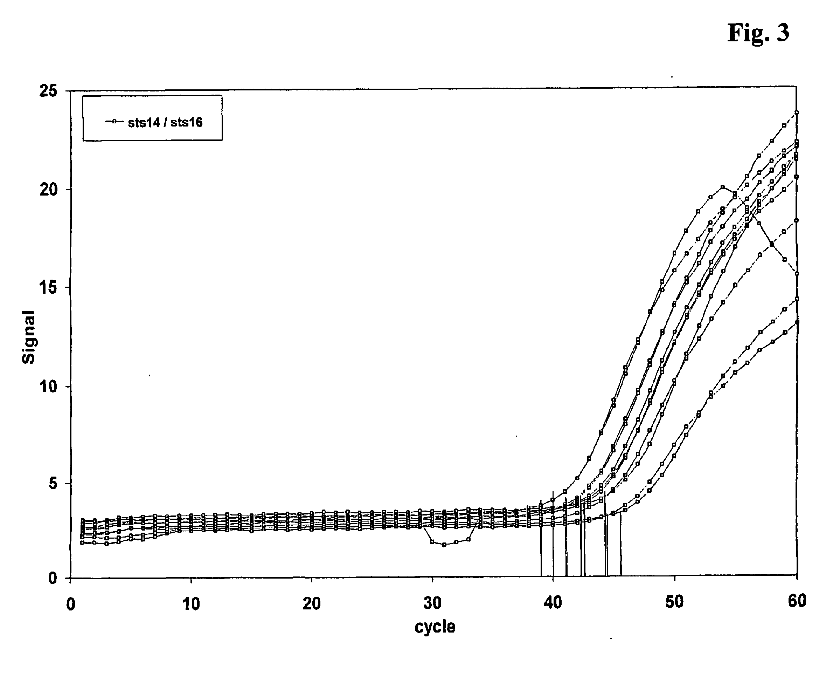

- **Figure 3**: Kinetic PCR growth curves for the primer combination STS14/16 along with probe STS15 basing on an eluate derived from a 1000 IU/mL sample. The vertical lines indicate when the growth curves cross the threshold, in other words, when an unambiguous and specific signal is initially detectable.

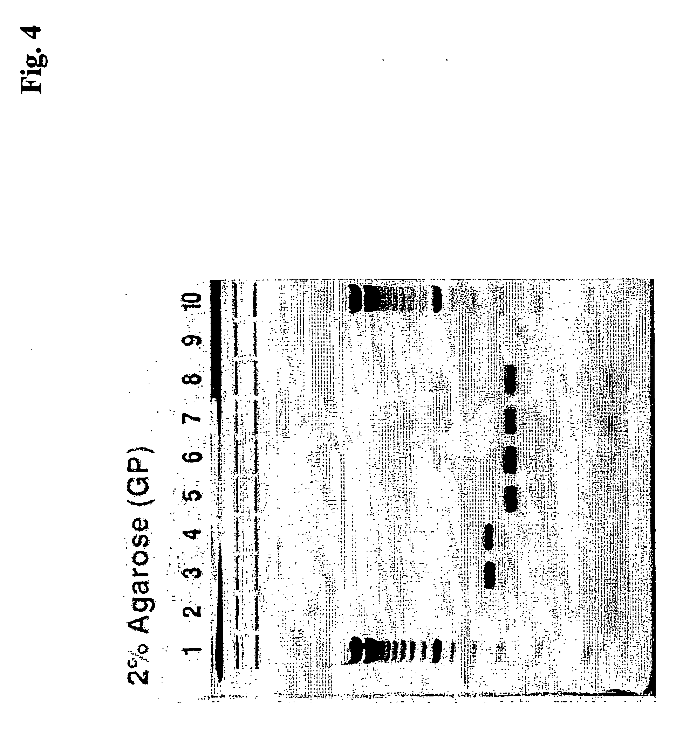

- **Figure 4**: Analysis of amplification products derived from the experiments in Example 1 by means of standard agarose gel electrophoresis (E-Gel system, Invitrogene, Carlsbad, CA, USA). (Lane 1: 100bp ladder DNA; 2: STS12/16: Water control; 3: STS12/16: 1000 IU/mL sample; 4: STS12/16: 1000 IU/mL sample; 5: STS13/16: 1000 IU/mL sample; 6: STS13/16: 1000 IU/mL sample; 7: STS14/16: 1000 IU/mL sample; 8: STS14/16: 1000 IU/mL sample; 9: empty; 10: 100bp ladder DNA)

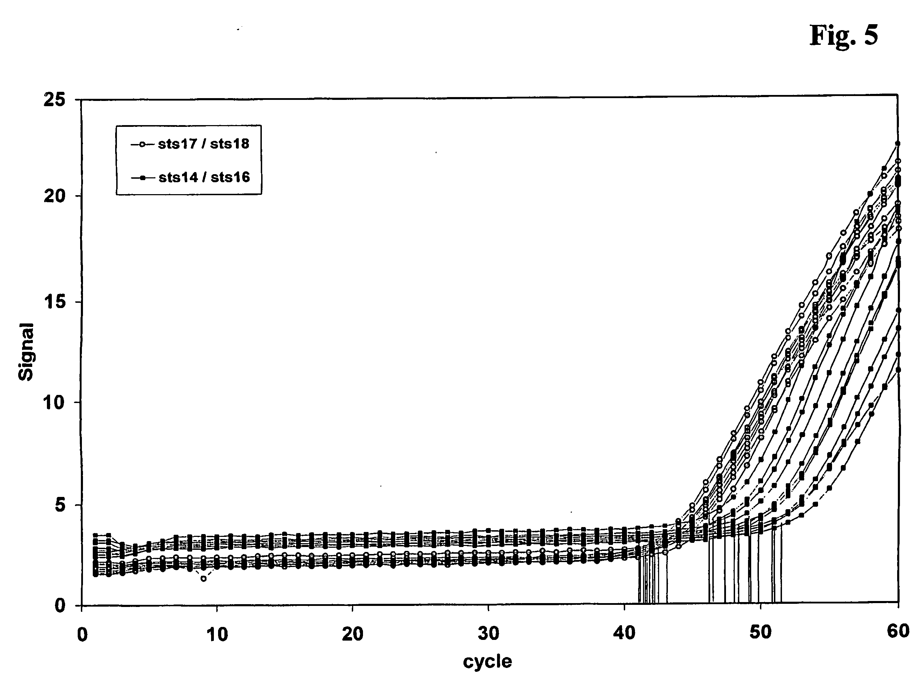

- **Figure 5**: Kinetic PCR growth curves for the primer combination STS14/16 compared to the primer combination STS17/18 along with probe STS15 basing on an eluate derived from a 1000 IU/mL sample. The vertical lines indicate when the growth curves cross the threshold, in other words, when an unambiguous and specific signal is initially detectable.

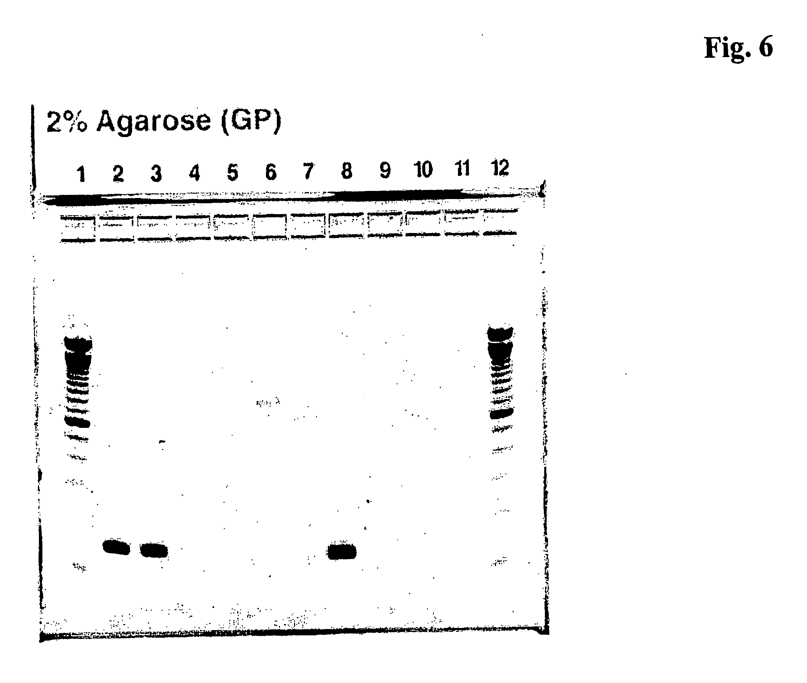

- **Figure 6**: Analysis of amplification products derived from the experiments in Example 2 by means of standard agarose gel electrophoresis (E-Gel system, Invitrogene, Carlsbad, CA, USA). (Lane 1: 100bp ladder DNA; 2: 50 IU/mL sample; PCR positive; 3: 25 IU/mL sample; PCR positive; 4: 25 IU/mL sample; PCR negative; 5: 10 IU/mL sample; PCR negative; 6: 10 IU/mL sample; PCR negatives 7: 10 IU/mL sample; PCR negative; 8: 10 IU/mL sample; PCR positive; 9: Negative Control; PCR negative; 10: Negative Controls PCR negative; 11: STS17/18: Water Control; PCR negatives 12: 100bp ladder DNA)

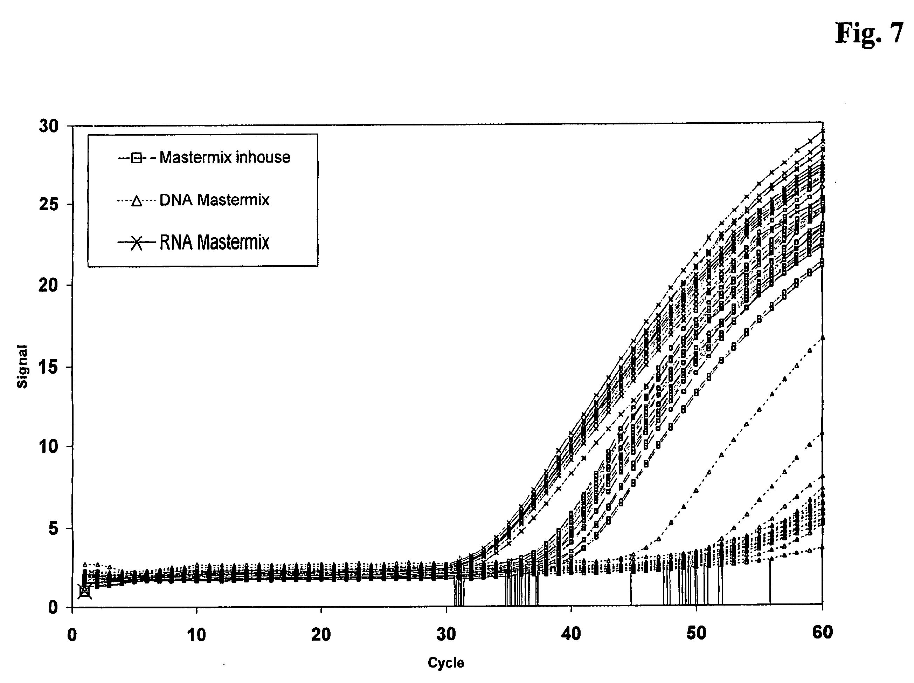

- **Figure 7**: Kinetic PCR growth curves for the primer combination STS17/18 along with probe STS15 basing on an eluate derived from a 1E+04= IU/mL sample. The vertical lines indicate when the growth curves cross the threshold, in other words, when an unambiguous and specific signal is initially detectable.

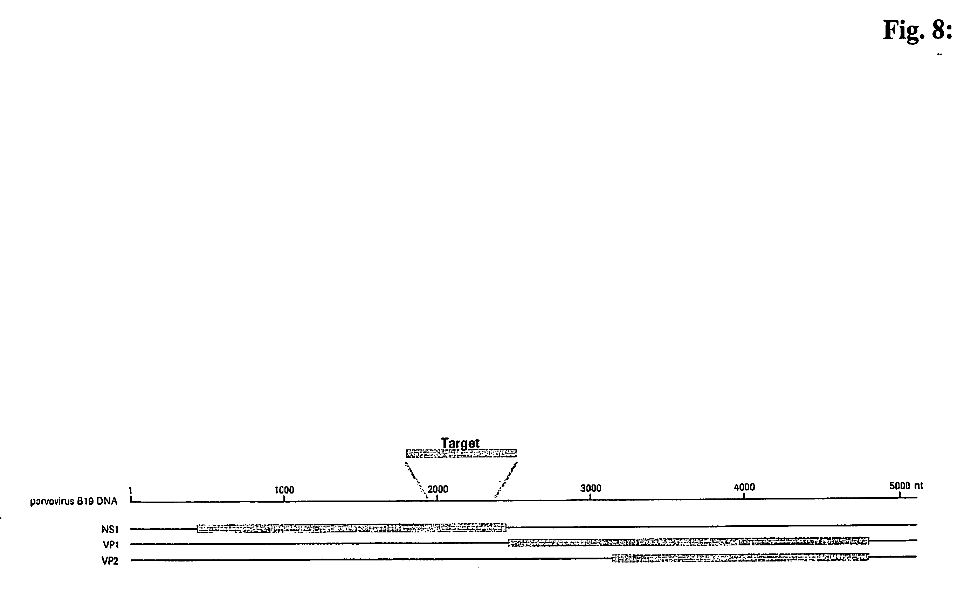

- **Figure 8**: Schematical drawing of the target region in the parvovirus B19 genome.

### Examples - Detection of parvovirus B19 by Nucleic acid testing

### General:

Sample preparation of EDTA plasma, citrate plasma and human serum has been performed employing the Total Nucleic Acid Isolation (TNAI) Kit on the COBAS AmpliPrep Instrument (both Roche Diagnostics GmbH, D-68305 Mannheim, Germany) following the manufacturer's instructions with 200 µL specimen input volume. 50 µL of eluate were then manually transferred to specific PCR tubes (referred to as k-tubes) and mixed with 50 µL of PCR reaction mix. Amplification and detection of nucleic acids was performed by kinetic PCR on the COBAS TaqMan Analyzer (Roche Diagnostics GmbH, D-68305 Mannheim, Germany) with the following PCR profile:

| **Step** | **Type** | **Ramp Slope** **(0.1 °C/s)** | **Temp.** **(°C)** | **Time** **(sec.)** | **Delay** **(sec.)** | **Number** **of Cycles** |
|---|---|---|---|---|---|---|
| 1 | Cover Heating | 3 | 100.0 | | | 1 |
| 2 | PreCycle | 12 | 50 | 300 | | 1 |
| 4 | PreCycle | 12 | 95 | 120 | | 1 |
| 5 | Sequence Start | | | | | |
| 6 | Denaturation | 12 | 95.0 | 15 | | |
| 7 | Annealing | 12 | 58 | 25 | 25 | |
| 8 | Sequence End | | | | | 60 |
| 9 | PostCycle | 12 | 40 | 120 | | 1 |

### Example 1

### Comparison of different Primer combinations

A TaqMan probe (STS15) comprising a highly conserved region of the NS 1 gene was designed. The probe has the sequence SEQ ID NO: 11 5'-CCCCGGGACCAGTTCAGGAGAATCAT-3' (nt 2070-2095 according to Shade R. O. et al., J. Virol. 58 (1986) 921-936). The probe has a melting temperature (Tm) of approx. 80 °C when the Nearest Neighbor Method is applied (OLIGO, Molecular Biology Insights, Inc, CO, USA). Next, primer sequences have been designed to fit the following criteria:
- Tm 59-63°C
- no or less mismatches with published Erythrovirus sequences incl. recently discovered new variants (Nguyen Q. T. et al., Virology 301 (2002) 374-80; Servant A. et al., J. Virol. 76 (2002) 9124-34)
- less false priming sites
- primer should end with A or C

The following sequences were deemed suitable and have been synthesized:
STS12 (fwd primer): 5'-GTGGTGAAAGCTCTGAAGAA-3' (SEQ ID NO: 12)
STS13 (fwd primer): 5'-GAAACCCCGCGCTCTA-3' (SEQ ID NO: 13)
STS14 (fwd primer): 5'-AAACCCCGCGCTCTAGTA-3' (SEQ ID NO: 14)
STS17 (fwd primer): 5'-GAAACCCCGCGCTCTAGTAC-3' (SEQ ID NO: 15)
STS16 (rev primer): 5'-TTCCATCCATTATACCAAGC-3' (SEQ ID NO: 16)
STS18 (rev primer): 5'-CCCAACTAACAGTTCACGAA-3' (SEQ ID NO: 17)

The performance of the primer combinations STS12/16, STS13/16, STS14/16, and STS17/18 was evaluated with the probe STS15 and a PCR reaction mix consisting of 50 mM Tricine pH 8.3, 100 mM potassium acetate, 3 mM manganese acetate, 4 % glycerol, 300 µM dATP, 300 µM dCTP, 300 µM dGTP, 50 µM dTTP, 500 µM dUTP, 10 U uracil-*N*-glycosylase, 40 U Z05 polymerase, 200 nM NTQ21-46A-Aptamer, 400 nM each primer and 100 nM probe. Samples consisting of 500 and 1000 IU/mL parvovirus B19 DNA were processed on COBAS AmpliPrep instrument using the TNAI Kit. Afterwards, 50 µL of the corresponding eluate were mixed with a 2 fold reaction mix in a k-tube and placed in the COBAS TaqMan Analyzer for Amplification/Detection.

Figures 1 through 3 show the kinetic PCR growth curves for the primer combinations STS12/16, STS13/16 and STS14/16 basing on an eluate derived from a 1000 IU/mL sample. The vertical lines indicate when the growth curves cross the threshold, in other words, when an unambiguous and specific signal is initially detectable. The crossing points of these vertical lines with the X axis are determined as cycle threshold (ct)-values and are directly proportional to the input concentration of the target sequence. The lower a ct-value the higher the initial input of target copies. From the here presented primer combinations STS14/16 appears to be superior as judged by the lower ct-values.

Figure 4 shows analysis of the corresponding amplicons by means of agarose gel electrophoresis. It demonstrates that the PCR reactions with the given primer combinations do exhibit none or less cross reactivity or amplification of unspecific sequences.

A second reverse primer, STS18, has been evaluated along with the forward primer STS17 and the primer combination has been compared with STS14/16 with a reaction mix and PCR protocol as described above. As samples served eluates derived from replicated extractions of a 500 IU/mL specimen. Figure 5 depicts a comparison of the two primer combinations. As evident from here, STS17/18 has been choosen for further experiments since it comprises the lowest ct-values along with less deviation on ct-value basis.

### Example 2

### Analytical Sensitivity with primers STS 17, STS18 and probe STS 15

For determination of sensitivity, dilution series of the World Health Organisation Standard for Parvovirus B19 DNA (National Institute for Biological Standards and Control [NIBSC] 1^{st} International Standard 2000 Parvovirus B19 DNA 500000 IU/mL; Code 99/800) from 1000-10 IU/mL in EDTA plasma were processed in twelve replicates using the extraction method described above with 200 µl specimen input volume. Fifty microliters of eluate were manually transferred to K-tubes, mixed with 50 µl activated MMx and subsequently taken for PCR (for details, see Example 1). According to the Probit analysis algorithm the sensitivity was found to be 26 IU/mL at 95 % hitrate. Table 1 summarizes the results of the study and depicts the hitrates at the corresponding input concentration. Figure 6 shows agarose gel eclectrophoresis analysis of selected amplification products.

**Table 1:**

| **NR** | **Concentration** **IU/mL** | **Number of** **Trials** | **Number** **of Positives** | **Hit rate** |
|---|---|---|---|---|
| 1 | 500 | 12 | 12 | 100% |
| 2 | 400 | 12 | 12 | 100% |
| 3 | 300 | 11 | 11 | 100% |
| 4 | 200 | 12 | 12 | 100% |
| 5 | 100 | 11 | 11 | 100% |
| 6 | 75 | 12 | 12 | 100% |
| 7 | 50 | 11 | 11 | 100% |
| 8 | 25 | 11 | 10 | 91% |
| 9 | 10 | 12 | 9 | 75% |

### Example 3

### Precision testing with different primer combinations

Precision with the primer combination STS17/18 has been evaluated in comparison to the combination STS14/16 along with probe STS15. The Pelispy Parvo-B19-DNA run control at 1E4 IU/mL (VQC Laboratory, Alkmaar, NL) has been extracted in 46 replicates on two different days following the procedure described above. The eluates have been analysed by kinetic PCR as described in Example 1. The overall CV on basis of the ct-values was found to be 2.18% in the case of the primer combination STS17/18 whereas the combination STS14/16 comprises a CV of 4.89% ( Table 2).

**Table 2:**

| | **ct-values** | |
|---|---|---|
| | Primer combination STS14/STS16 | Primer combination STS17/STS18 |
| Day 1 | 39,8 | 35,1 |
| | 40,0 | 33,5 |
| | 41,6 | 34,3 |
| | 40,6 | 35,7 |
| | 41,2 | 36,9 |
| | 40,0 | 35,0 |
| | 40,6 | 34,3 |
| | 42,0 | 34,8 |
| | 40,0 | 35,1 |
| | 41,0 | 35,5 |
| | 41,0 | 35,4 |
| | 41,3 | 35,4 |
| | 40,9 | 34,8 |
| | 42,5 | 35,4 |
| | 41,3 | 33,2 |
| | 43,4 | 34,4 |
| | 40,9 | 35,1 |
| | 42,4 | 36,0 |
| | 43,2 | 36,7 |
| | 42,1 | 35,8 |
| | 44,4 | 35,5 |
| | 41,8 | 35,3 |
| | 40,9 | 36,7 |
| Day 2 | 39,8 | 35,5 |
| | 40,2 | 35,1 |
| | 36,4 | 34,5 |
| | 36,3 | 35,7 |
| | 37,8 | 35,8 |
| | ND | 34,5 |
| | 38,4 | 35,2 |
| | 40,2 | 35,4 |
| | 39,5 | 34,7 |
| | 37,5 | 35,2 |
| | 40,5 | 35,5 |
| | 36,2 | 35,6 |
| | 38,4 | 35,3 |
| | 39,5 | 35,9 |
| | 37,2 | 35,6 |
| | 38,6 | 37,4 |
| | 37,7 | 35,2 |
| | 41,7 | 35,5 |
| | 44,3 | 35,8 |
| | 38,6 | 36,4 |
| | 40,1 | 35,5 |
| | 39,0 | 34,8 |
| | 41,4 | 35,0 |
| mean | 40,3 | 35,3 |
| SD | 1,968 | 0,770 |
| CV | 4,89 | 2,18 |

### Example 4

### Specificity testing with different primer combinations

Specificity has been evaluated with different primer combinations and probe STS15 located within the NS1 gene of the Parvovirus B19 genome. The general procedure (reaction mix, PCR protocol) was adapted from Example 1. Fresh routine blood donations were supplied by the Blood Donation Center of the German Red Cross, Munich, BRD, and have been extracted as described above. The following Table 3 depicts the results from this analysis and demonstrates a specifictity of 100% for all investigated combinations.

**Table 3:**

| **int. Nr.** | **BRK-Plasma Nr.** | **PCR Test with STS13/STS16** | **PCR Test with STS14/STS16** | **PCR Test with STS17/STS18** |
|---|---|---|---|---|
| 301 | 0926091 | negative | ND | ND |
| 302 | 0926033 | negative | negative | negative |
| 303 | 0926094 | negative | negative | negative |
| 304 | 0926037 | negative | negative | negative |
| 305 | 0926086 | negative | negative | negative |
| 306 | 0926038 | ND | ND | negative |
| 307 | 0926092 | ND | ND | negative |
| 308 | 0926049 | ND | ND | negative |
| 309 | 0926061 | ND | ND | negative |
| 310 | 0926068 | ND | ND | negative |
| 311 | 0926042 | ND | ND | negative |
| 312 | 0926076 | ND | negative | negative |
| 313 | 0926043 | negative | negative | negative |
| 314 | 0926073 | negative | negative | negative |
| 315 | 0926046 | negative | negative | negative |
| 316 | 0926072 | negative | negative | negative |
| 317 | 0926067 | negative | negative | negative |
| 318 | 0926050 | negative | negative | negative |
| 319 | 0926090 | ND | ND | negative |
| 320 | 0926079 | negative | negative | negative |
| 321 | 0926053 | negative | negative | negative |
| 322 | 0926051 | negative | negative | negative |
| 323 | 0926063 | negative | negative | negative |
| 324 | 0926088 | negative | negative | negative |
| 325 | 0926071 | negative | negative | ND |
| 326 | 0926034 | negative | negative | negative |
| 327 | 0926039 | negative | negative | negative |
| 328 | 0926087 | negative | negative | negative |
| 329 | 0926095 | negative | negative | negative |
| 330 | 0926036 | negative | negative | negative |
| 331 | 0926093 | negative | negative | negative |
| 332 | 0926064 | negative | negative | negative |
| 333 | 0926065 | negative | negative | negative |
| 334 | 0926085 | negative | negative | negative |
| 335 | 0926029 | negative | negative | negative |
| 336 | 0926032 | negative | negative | negative |
| 337 | 0926082 | ND | negative | negative |
| 338 | 0926098 | ND | negative | negative |
| 339 | 0926047 | negative | negative | negative |
| 340 | 0926097 | negative | negative | negative |
| 341 | 0941982 | negative | negative | negative |
| 342 | 0926069 | negative | negative | negative |
| 343 | 0926041 | negative | negative | negative |
| 344 | 0941983 | negative | negative | negative |
| 345 | 0926066 | negative | negative | negative |
| 346 | 0926035 | negative | negative | negative |
| 347 | 0926089 | negative | ND | negative |
| 348 | 0926045 | negative | ND | negative |
| 349 | 0926084 | ND | ND | ND |
| 350 | 0941971 | negative | negative | negative |
| 351 | 0926054 | negative | negative | negative |
| 352 | 0941962 | negative | negative | negative |
| 353 | 0926052 | ND | negative | negative |
| 354 | 0926081 | negative | negative | negative |
| 355 | 0941985 | negative | negative | negative |
| 356 | 0926080 | negative | negative | negative |
| 357 | 0937866 | negative | negative | negative |
| 358 | 0937882 | negative | negative | negative |
| 359 | 0937864 | negative | negative | negative |
| 360 | 0937861 | negative | negative | negative |

### Example 5

### Influence of reaction mix composition on PCR precision

Goal of the experiment was to analyse whether the reaction mix composition has a significant influence on the precision of the PCR reaction targeting the Parvovirus B19 NS1 region. The primer combination STS17/18 (0.4 µM final concentration each) and the probe STS15 (0.1 µM final concentration) have been added to the reaction mix described in Example 4 as well as to two preformulated reaction mixes which are commercially available (COBAS TaqMan Generic DNA Amplification Kit, COBAS TaqMan Generic RNA Amplification Kit). The Pelispy Parvo-B19-DNA run control at 1E4 IU/mL as described in Example 3 has been extracted in 20 replicates per reaction mix and analysed according to Example 1. Figure 7 demonstrates that the commercial RNA reaction mix comprises the lowest ct-values along with the best precision as judged by lowest CV of 0.5% compared to the commercial DNA reaction mix with a CV of 4,4% and the inhouse reaction mix with a CV of 2.3% (Table 4).

A survey of the target region within the genome of parvovirus B19 is shown in Fig. 8.

**Table 4:**

| | **ct-values with primer combination STS17/STS18** | | |
|---|---|---|---|
| | inhouse reaction mix | commercial generic RNA reaction mix | commercial generic DNA reaction mix |
| | 35,2 | 31,2 | 47,7 |
| | 35,5 | 31,3 | 47,4 |
| | 35,2 | 31,1 | 49,1 |
| | 35,8 | 31,1 | 49,2 |
| | 34,9 | 30,8 | 50,6 |
| | 35,3 | 31,1 | 48,6 |
| | 36,0 | 30,6 | 44,8 |
| | 34,8 | 31,1 | 51,8 |
| | 34,7 | 31,2 | 50,1 |
| | 35,2 | 31,2 | 47,9 |
| | 35,1 | 31,2 | 49,6 |
| | 36,3 | 31,2 | 48,6 |
| | 37,3 | 31,0 | 52,1 |
| | 35,5 | 31,0 | 50,9 |
| | 34,9 | 31,1 | 50,1 |
| | 35,7 | 31,1 | 49,9 |
| | 37,3 | 31,2 | 55,9 |
| | 35,9 | 31,2 | 52,1 |
| | 37,2 | 31,1 | 48,9 |
| | 36,6 | 31,4 | 49,4 |
| mean | 35,7 | 31,1 | 49,7 |
| SD | 0,811 | 0,166 | 2,191 |
| CV | 2,27 | 0,53 | 4,41 |

### List of References

Abramson R. D. and Myers T. W., Curr Opin Biotechnol 4 (1993) 41-47
Alderton R. P. et al., S., Anal. Biochem. 201 (1992) 166-169
Anderson M. J., Lancet 1 (1983) 1378
Astell et al., J. Gen. Virol. 68 (1987) 885-893
Ausubel F. et al.: Current Protocols in Molecular Biology 1987, J. Wiley and Sons, NY
Barany F., PCR Methods and Applic.1 (1991) 5-16
Barany F., Proc. Natl. Acad. Sci. USA 88 (1991)189-193
Beaucage et al., Tetrahedron Letters 22 (1981) 1859
Boom R., J Clin Microbiol 28 (1990) 495-503
Brown E. L., et al., Methods in Enzymology 68 (1979) 109-151
Brown K. E., Baillieres Best Pract Res Clin Haematol 13 (2000) 245-259
Carriere C. et al., J Virol Methods 44 (1993), 221-234
Cohen B., BMJ 311 (1995) 1549-1552
Cotmore S. F. et al., J. Virol. 60 (1986) 548-557
Cubie H. A. et al., Mol Cell Probes 9 (1995), 59-66
DE 3724442
DE 3734442
Dorsch S. et al., J. Gen. Virol. (2001) 82, 191-199
Dux S. et al., Dtsch Med Wochenschr 127 (2002) 1584-1588
Enders E: Infections of the fetus and the neonate other than rubella. Topley & Wilson's Microbiology and Microbial Infections. Edited by Collier L. London, Edward Arnold, 1998, pp. 873-915
Enders G. et al., Clin Infect Dis 26 (1998) 355-358
EP 0439182
EP 238 893
EP 783 580
Erdman D. D. et al., J Gen Virol 77 (1996), 2767-2774
Garegg et al., Chem. Scr. 25 (1985) 280-282
GB 91/00212
Guatelli J.C., et al., Proc. Natl. Acad. Sci. USA 87 (1990) 1874-1878
Hemauer A. et al., J. Gen. Virol. (1996) 77, 1781-1785
Hicks K. E. et al., Arch Virol. 141 (1996), 1319-1327
Holloway B. et al., Nucleic Acids Res 21 (1993), 3905-3906
Jakobi R. et al., Anal. Biochem. 175 (1988) 196-201
Jordan J. A. et al., Mol. Diagn. 1 (1996), 321-328
JP 04088985
JP 11221099
JP 147986/1995
Jungkind D., J Clin Virol 20 (2001) 1-6
Kwoh D. Y. et al., Proc. Natl. Acad. Sci. USA 86 (1989) 1173-1177
Marko M. A. et al., Anal. Biochem. 121 (1982) 382-387
Narang S. A. et al., Methods in Enzymology 68 (1979) 90-98
Nguyen Q. T. et al., Virology 301 (2002) 374-80
Oligonucleotide Synthesis, Gait, M.J., ed., 1984; Nucleic Acid Hybridization, Hames, B.D., and Higgins, S.J., eds., 1984; and a series, Methods in Enzymology, Academic Press, Inc., all of which are incorporated herein by reference
Ozawa K. et al., J. Virol. 62 (1988) 2508-2511
Pattison J. R. et al., Lancet 1 (1981) 664-665
RU2146372
Sambrook J. et al., Molecular Cloning: A Laboratory Manual, Cold Spring Harbor Laboratory Press, Cold Spring Harbor, New York, 1989
Sato K. et al., J Clin Microbiol 38 (2000) 1241-1243
Schorling S. et al., J. Mol. Diagn. 6 (2004) 37-41
Servant A. et al., J. Virol. 76 (2002) 9124-34
Shade R. O. et al., J. Virol. 58 (1986) 921-936
Stelzl E. et al., J Clin Microbiol 40 (2002) 1447-1450
Takahashi N. et al., FEBS Lett. 450 (1999) 289-293
Turton J. et al., Epidemiol Infect 105 (1990) 197-201
Uhlmann and Peyman, Chemical Reviews 90 (1990) 543
Umene K. and Nunoue T., J. Gen. Virol. 76 (1995) 2645-2651
US 4,458,066
US 4,996,143
US 5,130,238
US 5,210,015
US 5,386,024
US 5,478,972
US 5,487,972
US 5,565,322
US 5,585,254
US 5,804,375
US 5,849,489
US 6,103,476
US 6,162,603
US 6,174,670
US 6,183,999
US 6,268,349
US 6,274,307
Vassias I. et al., J. Virol. Meth. 44 (1993) 329-338
Verma S., and Eckstein F., Annu. Rev. Biochem. 67 (1998) 99-134
Vogelstein B. et al., Proc. Natl. Acad. USA 76 (1979) 615-9
Walsh, 1979, Enzymatic Reaction Mechanisms. W. H. Freeman and Company, San Francisco, Chapter 3
Whelen A. C. and Persing D. H., Annu. Rev. Microbiol. 50 (1996) 349-373
WO 00/24917
WO 01/06019
WO 01/14593
WO 01/37291
WO 02/00924
WO 02/096925
WO 02/12263
WO 03/002753
WO 90/01069
WO 91/04330
WO 92/08808
WO 96/41811
WO 98/04730
WO 99/ 16781
WO 99/28439
Wu D. Y. and Wallace R. B., Genomics 4 (1989) 560-69
Yoto Y. et al., Lancet 347 (1996) 868-9

## Claims

1. A method for the detection of a target nucleic acid comprising the nucleic acid sequence of parvovirus B19 in a sample comprising the steps of
(a) providing a sample suspected to contain the target nucleic acid
(b) providing a pair of primers comprising a first and a second primer wherein the first primer consists of nucleic acid sequence SEQ ID NO: 15 and wherein the second primer consists of of nucleic add sequence SEQ ID NO: 17,
(c) amplifying the target nucleic acid,
(d) detecting the amplified target nucleic acid of step (c).

2. The method according to claim 1, additionally comprising, between steps (c) and (d)
(c1) contacting the sample with a probe under conditions for binding the probe to the target nucleic acid,
and wherein step (d) comprises
detecting the binding product between the target nucleic acid and the probe as an indication of the presence of the target nucleic acid.

3. The method according to claim 2, wherein the probe consists of at least 12 contiguous nucleotides of the nucleic acid sequence SEQ ID NO: 5 or a complementary sequence thereof.

4. The method according to any of the claims 2 or 3 wherein the probe carries a labeL

5. A method according to claim 4 wherein an additional probe carrying a label is contacted with the sample in step d) so that a pair of probes consisting of a first and a second probe is contacted with the sample in step d).

6. A method according to claim 5 wherein said amplifying step c) comprises contacting the sample with the said pair of primers to produce an amplification product if the target nucleic acid is present in said sample, wherein said hybridizing step d) comprises contacting said sample with the pair of probes, wherein the members of said pair of probes hybridize to said amplification product within no more than five nucleotides of each other, wherein the first probe of said pair of probes is labeled with a donor fluorescent label and wherein the second probe of said pair of probes is labeled with a corresponding acceptor fluorescent label;
and detecting the binding product between the target nucleic acid and the pair of probes in step e) by detecting the presence or absence of fluorescence resonance energy transfer between said donor fluorescent label of said first probe and said acceptor fluorescent label of said second probe, wherein the presence of fluorescence resonance energy transfer is indicative of the presence of the target nucleic acid in the sample, and wherein the absence of fluorescence resonance energy transfer is indicative of the absence of the target nucleic acid in the sample.

7. The method according to any of the claims 2 or 3 wherein the probe carries a first and a second label.

8. The method according to any of the claims 2 to 7, wherein the target nucleic in step c) is amplified with a template-dependent DNA polymerase.

9. The method according to any of the claims 7 to 8 whereby the binding product between the target nucleic acid and the probe in step (e) is detected by the quantity of the first or second fluorescent label that is released from the probe hybridized to the target nucleic acid by exonuclease hydrolysis by the template-dependent DNA polymerase.

10. A method according to any of the claims 2 to 9 wherein the probe consists of at least 12 contiguous nucleotides of the nucleic acid sequence SEQ ID NO: 10 or a complementary sequence thereof.

11. A method according to claim any of the claims 2 to 10 wherein the probe has the nucleic acid sequence SEQ ID NO: 11 or a complementary sequence thereof.

12. A method according to any of the claims 2 to 11 wherein the primer and/ or the probe comprise a modified nucleotide or a non-nucleotide compound.

13. A method According to any of the claims 2 to 12 wherein other target nucleic acids are detected in the same reaction.

14. A method according to claim 13 wherein the other target nucleic acids comprise nucleic acid from hepatitis A virus, hepatitis B virus, hepatitis C virus, west nile virus or the human immunodeficiency virus.

15. A pair of primers comprising a first and a second primer whereby the nucleic acid sequence of the first primer is SEQ ID NO: 15 and whereby the nucleic acid sequence of the second primer is SEQ ID NO: 17.

16. Use of a pair of primers according to claim 15 in a hybridisation reaction with a complementary nucleic acid.

17. A kit comprising a template dependent DNA polymerase, nucleotides and a pair of primers according to claim 15.

## Patentansprüche

1. Verfahren zum Nachweis einer Zielnukleinsäure, die die Nukleinsäuresequenz von Parvovirus B19 umfasst, in einer Probe, mit den folgenden Schritten:
(a) Bereitstellen einer Probe, die vermutlich die Zielnukleinsäure enthält;
(b) Bereitstellen eines Primerpaars, das einen ersten und einen zweiten Primer umfasst, wobei der erste Primer aus der Nukleinsäuresequenz SEQ ID NO: 15 besteht und wobei der zweite Primer aus der Nukleinsäuresequenz SEQ ID NO: 17 besteht;
(c) Amplifizieren der Zielnukleinsäure;
(d) Nachweisen der amplifizierten Zielnukleinsäure aus Schritt (c).

2. Verfahren nach Anspruch 1, mit dem zusätzlichen Schritt zwischen den Schritten (c) und (d):
(c1) Inkontaktbringen der Probe mit einer Sonde unter Bedingungen für die Bindung der Sonde an die Zielnukleinsäure;
und wobei Schritt (d)
das Nachweisen des Bindungsprodukts zwischen der Zielnukleinsäure und der Sonde als Hinweis auf das Vorhandensein der Zielnukleinsäure umfasst.

3. Verfahren nach Anspruch 2, wobei die Sonde aus wenigstens 12 zusammenhängenden Nukleotiden der Nukleinsäuresequenz SEQ ID NO: 5 oder einer komplementären Sequenz davon besteht.

4. Verfahren nach einem der Ansprüche 2 oder 3, wobei die Sonde eine Markierung trägt.

5. Verfahren nach Anspruch 4, wobei eine eine Markierung tragende zusätzliche Sonde mit der Probe in Schritt d) in Kontakt gebracht wird, so dass ein Sondenpaar, das aus einer ersten und einer zweiten Sonde besteht, mit der Probe in Schritt d) in Kontakt gebracht wird.

6. Verfahren nach Anspruch 5, wobei der Amplifizierungsschritt c) das Inkontaktbringen der Probe mit dem Primerpaar umfasst, so dass ein Amplifikationsprodukt entsteht, wenn die Zielnukleinsäure in der Probe vorliegt, wobei der Hybridisierungsschritt d) das Inkontaktbringen der Probe mit dem Sondenpaar umfasst, wobei die Mitglieder des Sondenpaars an das Amplifikationsprodukt in einem Abstand von höchstens fünf Nukleotiden zueinander hybridisieren, wobei die erste Sonde des Sondenpaars mit einer Donor-Fluoreszenzmarkierung markiert ist und wobei die zweite Sonde des Sondenpaars mit einer entsprechenden Akzeptor-Fluoreszenzmarkierung markiert ist;
und Nachweisen des Bindungsprodukts zwischen der Zielnukleinsäure und dem Sondenpaar in Schritt e), indem das Vorliegen oder Fehlen eines Fluoreszenzresonanz-Energietransfers zwischen der Donor-Fluoreszenzmarkierung der ersten Sonde und der Akzeptor-Fluoreszenzmarkierung der zweiten Sonde nachgewiesen wird, wobei das Vorliegen eines Fluoreszenzresonanz-Energietransfers das Vorhandensein der Zielnukleinsäure in der Probe anzeigt und wobei das Fehlen eines Fluoreszenzresonanz-Energietransfers die Abwesenheit der Zielnukleinsäure in der Probe anzeigt.

7. Verfahren nach einem der Ansprüche 2 oder 3, wobei die Sonde eine erste und eine zweite Markierung trägt.

8. Verfahren nach einem der Ansprüche 2 bis 7, wobei die Zielnukleinsäure in Schritt c) mit einer matrizenabhängigen DNA-Polymerase amplifiziert wird.

9. Verfahren nach einem der Ansprüche 7 bis 8, wodurch das Bindungsprodukt zwischen der Zielnukleinsäure und der Sonde in Schritt (e) über die Menge der ersten oder zweiten Fluoreszenzmarkierung, die aus der an die Zielnukleinsäure hybridisierten Sonde durch Exonuklease-Hydrolyse durch die matrizenabhängige DNA-Polymerase freigesetzt wird, nachgewiesen wird.

10. Verfahren nach einem der Ansprüche 2 bis 9, wobei die Sonde aus wenigstens 12 zusammenhängenden Nukleotiden der Nukleinsäuresequenz SEQ ID NO: 10 oder einer komplementären Sequenz davon besteht.

11. Verfahren nach einem der Ansprüche 2 bis 10, wobei die Sonde die Nukleinsäuresequenz SEQ ID NO: 11 oder eine komplementäre Sequenz davon aufweist.

12. Verfahren nach einem der Ansprüche 2 bis 11, wobei der Primer und/oder die Sonde ein modifiziertes Nukleotid oder eine Nicht-Nukleotidverbindung umfassen.

13. Verfahren nach einem der Ansprüche 2 bis 12, wobei andere Zielnukleinsäuren in der gleichen Reaktion nachgewiesen werden.

14. Verfahren nach Anspruch 13, wobei die anderen Zielnukleinsäuren Nukleinsäure aus Hepatitis-A-Virus, Hepatitis-B-Virus, Hepatitis-C-Virus, West-Nile-Virus oder dem menschlichen Immunschwächevirus umfassen.

15. Primerpaar, das einen ersten und einen zweiten Primer umfasst, womit es sich bei der Nukleinsäuresequenz des ersten Primers um SEQ ID NO: 15 handelt und womit es sich bei der Nukleinsäuresequenz des zweiten Primers um SEQ ID NO: 17 handelt.

16. Verwendung eines Primerpaars nach Anspruch 15 in einer Hybridisierungsreaktion mit einer komplementären Nukleinsäure.

17. Kit, umfassend eine matrizenabhängige DNA-Polymerase, Nukleotide und ein Primerpaar nach Anspruch 15.

## Revendications

1. Procédé pour la détection d'un acide nucléique cible comprenant la séquence d'acides nucléiques du parvovirus B19 dans un échantillon, comprenant les étapes :
(a) de procuration d'un échantillon suspecté de contenir l'acide nucléique cible ;
(b) de procuration d'une paire d'amorces comprenant une première et une deuxième amorce, la première amorce étant constituée de la séquence d'acides nucléiques SEQ ID NO: 15 et la deuxième amorce étant constituée de la séquence d'acides nucléiques SEQ ID NO: 17 ;
(c) d'amplification de l'acide nucléique cible ;
(d) de détection de l'acide nucléique cible amplifié de l'étape (c).

2. Procédé selon la revendication 1, comprenant en outre, entre les étapes (c) et (d) :
(c1) la mise en contact de l'échantillon avec une sonde dans des conditions appropriées pour lier la sonde à l'acide nucléique cible ;
et dans lequel l'étape (d) comprend :
la détection du produit de liaison entre l'acide nucléique cible et la sonde comme indication de la présence de l'acide nucléique cible.

3. Procédé selon la revendication 2, dans lequel la sonde est constituée par au moins 12 nucléotides contigus de la séquence d'acides nucléiques SEQ ID NO: 5 ou d'une de ses séquences complémentaires.

4. Procédé selon l'une quelconque des revendications 2 ou 3, dans lequel la sonde porte un marqueur.

5. Procédé selon la revendication 4, dans lequel une sonde supplémentaire portant un marqueur est mise en contact avec l'échantillon à l'étape d), de telle sorte que l'on met une paire de sonde constituée par une première et une deuxième sonde en contact avec l'échantillon à l'étape d).

6. Procédé selon la revendication 5, dans lequel ladite étape d'amplification c) comprend la mise en contact de l'échantillon avec ladite paire d'amorces pour obtenir un produit d'amplification lorsque l'acide nucléique cible est présent dans ledit échantillon, ladite étape d'hybridation d) comprenant la mise en contact dudit échantillon avec la paire de sondes, les membres de ladite paire de sondes s'hybridant audit produit d'amplification à concurrence d'un nombre de nucléotides réciproques qui n'est pas supérieur à cinq, la première sonde de ladite paire de sondes étant marquée avec un marqueur fluorescent faisant office de donneur et la deuxième sonde de ladite paire de sonde étant marquée avec un marqueur fluorescent correspondant faisant office d'accepteur ;
et la détection du produit de liaison entre l'acide nucléique cible et la paire de sondes à l'étape e) en détectant la présence ou l'absence de fluorescence résultant du transfert de l'énergie de résonance entre ledit marqueur fluorescent faisant office de donneur de ladite première sonde et ledit marqueur fluorescent faisant office d'accepteur de ladite deuxième sonde, la présence de fluorescence résultant du transfert de l'énergie de résonance indiquant la présence de l'acide nucléique cible dans l'échantillon, et l'absence de fluorescence résultant du transfert de l'énergie de résonance indiquant l'absence de l'acide nucléique cible dans l'échantillon.

7. Procédé selon l'une quelconque des revendications 2 ou 3, dans lequel la sonde porte un premier et un deuxième marqueur.

8. Procédé selon l'une quelconque des revendications 2 à 7, dans lequel l'acide nucléique cible à l'étape c) est amplifié avec une ADN polymérase dépendant de la matrice.

9. Procédé selon l'une quelconque des revendications 7 à 8, dans lequel le produit de liaison entre l'acide nucléique cible et la sonde à l'étape (e) est détecté par la quantité du premier ou du deuxième marqueur fluorescent qui est libérée de la sonde hybridée à l'acide nucléique cible via une hydrolyse par exonucléase par l'ADN polymérase dépendant de la matrice.

10. Procédé selon l'une quelconque des revendications 2 à 9, dans lequel la sonde est constituée par au moins 12 nucléotides contigus de la séquence d'acides nucléiques SEQ ID NO: 10 ou d'une de ses séquences complémentaires.

11. Procédé selon l'une quelconque des revendications 2 à 10, dans lequel la sonde possède la séquence d'acides nucléiques SEQ ID NO: 11 ou une de ses séquences complémentaires.

12. Procédé selon l'une quelconque des revendications 2 à 11, dans lequel l'amorce et/ou la sonde comprennent un nucléotide modifié ou un composé non nucléotidique.

13. Procédé selon l'une quelconque des revendications 2 à 12, dans lequel d'autres acides nucléiques cibles sont détectés dans la même réaction.

14. Procédé selon la revendication 13, dans lequel les autres acides nucléiques cible comprennent des acides nucléiques du virus de l'hépatite A, du virus de l'hépatite B, du virus de l'hépatite C, du virus du Nil occidental ou du virus de l'immunodéficience humaine.

15. Paire d'amorces comprenant une première et deuxième amorce, la séquence d'acides nucléiques de la première amorce représentant la SEQ ID NO: 15 et la séquence d'acides nucléiques de la deuxième amorce représentant la SEQ ID NO: 17.

16. Utilisation d'une paire d'amorces selon la revendication 15, dans une réaction d'hybridation avec un acide nucléique complémentaire.

17. Nécessaire comprenant une ADN polymérase dépendant de la matrice, des nucléotides et une paire d'amorces selon la revendication 15.
